# EUROPEAN PATENT APPLICATION

(11) **EP 4 541 190 A1**
(43) Date of publication of application: **23.04.2025**
(21) Application number: 23826878.3
(22) Date of filing: 24.05.2023
(51) Int. Cl.: A01N 63/20, A01P 3/00, C12N 1/20, C12N 1/21, C12N 15/09, C12N 15/113, C12R 1/89

(54) **AGENT FOR INDUCING PLANT DISEASE RESISTANCE, METHOD FOR INDUCING PLANT DISEASE RESISTANCE, AND METHOD FOR PRODUCING AGENT FOR INDUCING PLANT DISEASE RESISTANCE**

(30) Priority: 20.06.2022 JP 2022099144
(71) Applicant: Panasonic Intellectual Property Management Co., Ltd., Kadoma-shi, Osaka 571-0057 (JP)
(72) Inventor: KOJIMA Seiji, Kadoma-shi, Osaka 571-0057 (JP)
(74) Representative: Grünecker Patent- und Rechtsanwälte PartG mbB
(86) International application number: PCT/JP2023/019242
(87) International publication number: WO 2023/248690

(57) **Abstract**

The present disclosure provides a plant disease resistance inducing agent that can effectively induce resistance to a plant disease. A plant disease resistance inducing agent according to the present disclosure contains a secretion product of a cyanobacterium.

## Description

### Technical Field

The present disclosure relates to a plant disease resistance inducing agent that is a natural metabolite contributing to plant disease resistance induction, a plant disease resistance inducing method, and a method for producing a plant disease resistance inducing agent.

### Background Art

There is a need for a technological development for efficiently producing high-quality agricultural crops in a limited arable land in response to a demand for an increase in food production due to an increase in the world population. More specifically, it is desired to shorten the cultivation period by promoting growth, to increase the harvest amount per cultivation area, to improve the yield by reducing diseases and non-standard products, and to increase the weight of fruits and improve the quality of products by increasing the sugar content or the like.

Furthermore, from the perspective of global warming mitigation and environmental load reduction, the use of biological raw materials for phasing out fossil resources is increasing. In particular, it is desired to utilize a naturally occurring material that consumes a small amount of fossil energy in a production process and has a small environmental load in application.

For example, a specific oxo fatty acid derivative (Patent Literature 1), a polypeptide chain forming part of elongation factor Tu (EF-Tu) (Patent Literature 2), and the like are known as naturally occurring materials that induce plant disease resistance. Furthermore, for example, it is known that plant disease resistance can be induced by inoculating a plant with a specific microorganism species (Patent Literature 3).

### Citation List

### Patent Literature

PTL 1: Japanese Patent No. 6759448
PTL 2: Japanese Unexamined Patent Application Publication No. 2015-077116
PTL 3: Japanese Patent No. 4359619

### Summary of Invention

### Technical Problem

However, in the related art, for example, a production process of a plant disease resistance inducer by a microorganism, a purification or extraction process of a disease resistance inducer, and the like are complicated, take a lot of time and effort, and entail high costs. Furthermore, inoculating a plant with a microorganism itself has different effects depending on the combination of the species of the microorganism used, the species of the target plant, and the properties of soil, thus lacking versatility and having unstable disease resistance induction effects.

Accordingly, the present disclosure provides a plant disease resistance agent that can effectively induce plant disease resistance, and a plant disease resistance inducing method. The present disclosure also provides a method for producing a plant disease resistance inducing agent, by which the plant disease resistance inducing agent can be simply and efficiently produced.

### Solution to Problem

A plant disease resistance inducing agent according to one aspect of the present disclosure contains a secretion product of a cyanobacterium.

### Advantageous Effects of Invention

A plant disease resistance inducing agent and a plant disease resistance inducing method according to the present disclosure can effectively induce plant disease resistance. A method for producing a plant disease resistance inducing agent according to the present disclosure can simply and efficiently produce a plant disease resistance inducing agent. Brief Description of Drawings

[Fig. 1] Fig. 1 is a flow chart of an example of a method for producing a plant disease resistance inducing agent according to an embodiment.
[Fig. 2] Fig. 2 is a view schematically illustrating a cell surface layer of a cyanobacterium.
[Fig. 3] Fig. 3 is a transmission electron micrograph of an ultrathin section of a modified cyanobacterium of Example 1.
[Fig. 4] Fig. 4 is a magnified image of a broken-line region A in Fig. 3.
[Fig. 5] Fig. 5 is a transmission electron micrograph of an ultrathin section of a modified cyanobacterium of Example 2.
[Fig. 6] Fig. 6 is a magnified image of a broken-line region B in Fig. 5.
[Fig. 7] Fig. 7 is a transmission electron micrograph of an ultrathin section of a modified cyanobacterium of Comparative Example 1.
[Fig. 8] Fig. 8 is a magnified image of a broken-line region C in Fig. 7.
[Fig. 9] Fig. 9 is a graph of the amount of protein in culture supernatants of the modified cyanobacteria of Example 1, Example 2, and Comparative Example 1 (n = 3, error bar = SD).

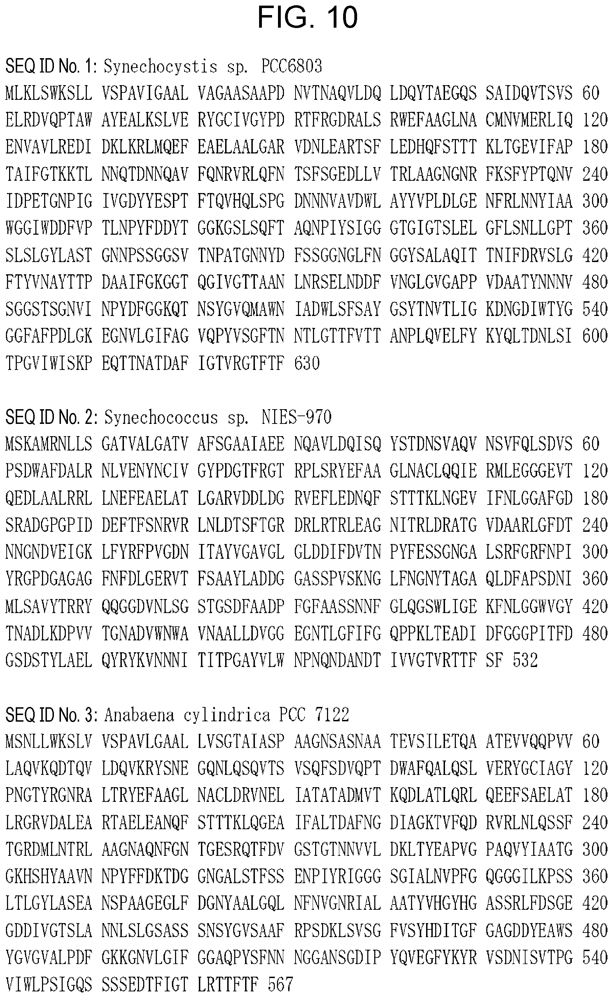

[Fig. 10] Fig. 10 shows SEQ ID No. 01 to SEQ ID No. 03.

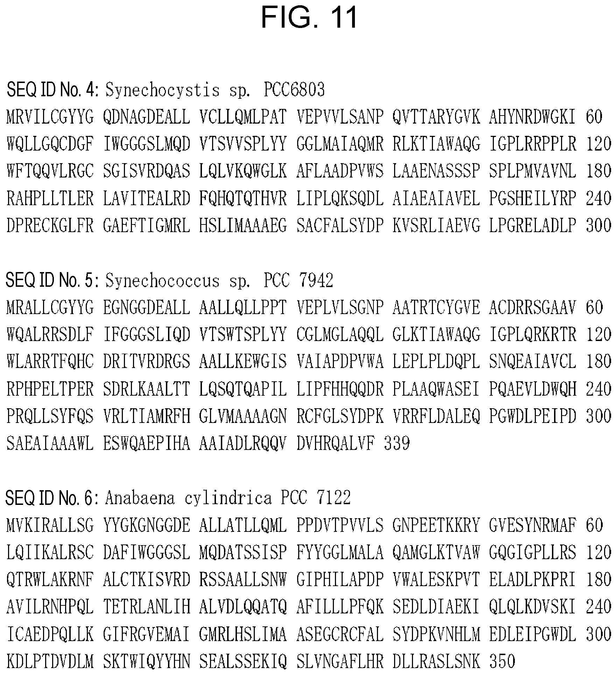

[Fig. 11] Fig. 11 shows SEQ ID No. 04 to SEQ ID No. 06.

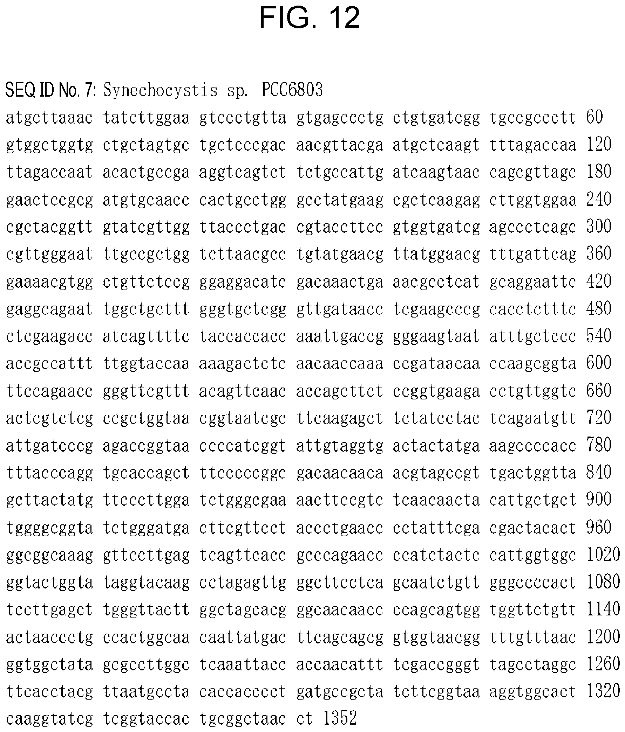

[Fig. 12] Fig. 12 shows SEQ ID No. 07.

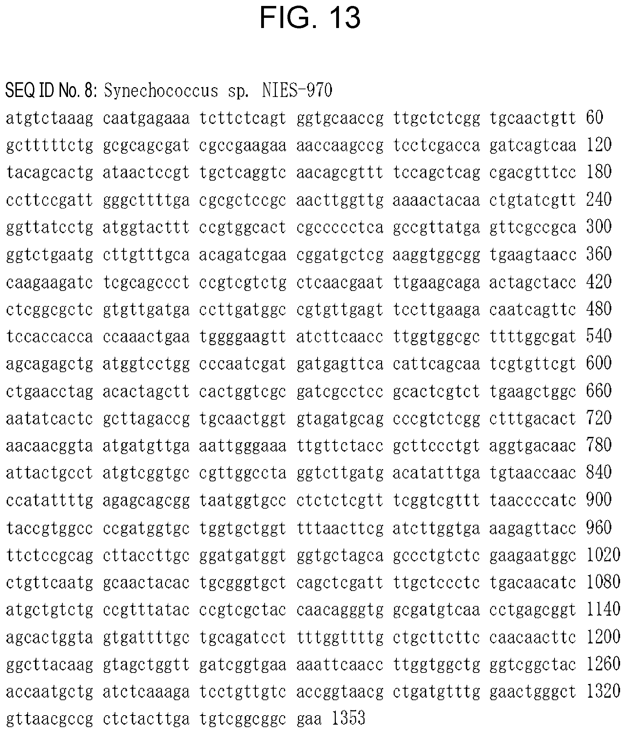

[Fig. 13] Fig. 13 shows SEQ ID No. 08.

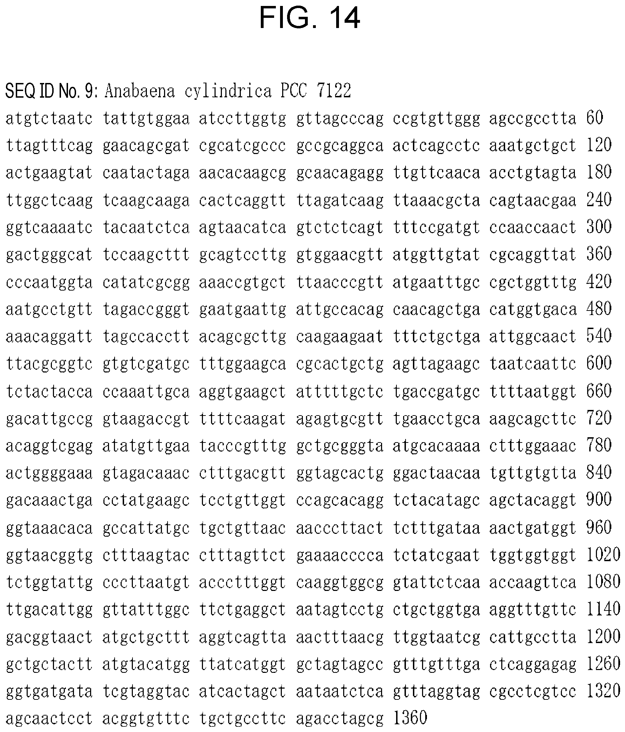

[Fig. 14] Fig. 14 shows SEQ ID No. 09.

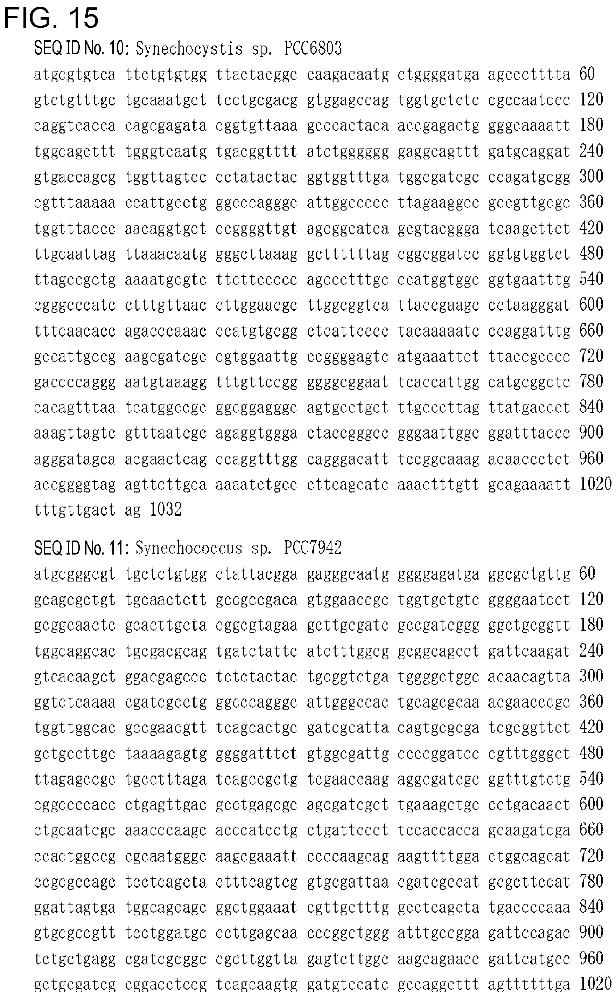

[Fig. 15] Fig. 15 shows SEQ ID No. 10 to SEQ ID No. 11.

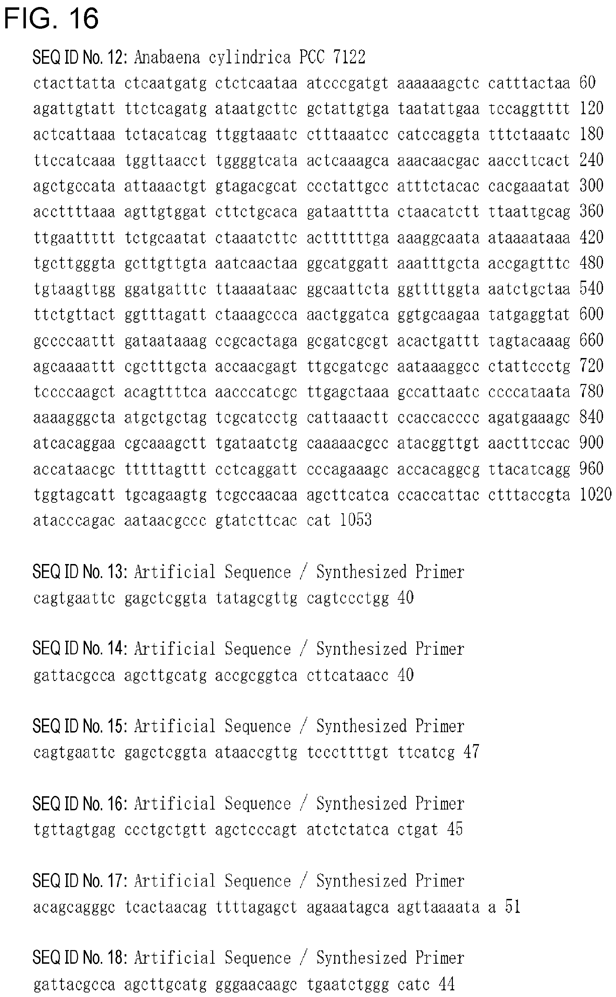

[Fig. 16] Fig. 16 shows SEQ ID No. 12 to SEQ ID No. 18.

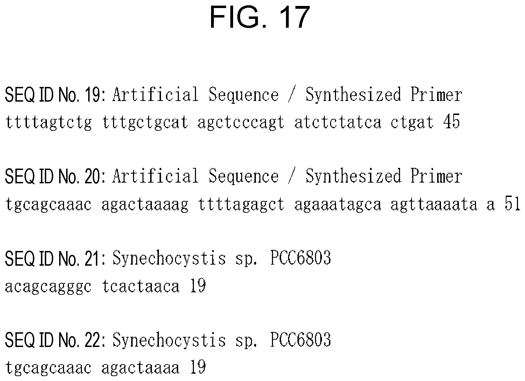

[Fig. 17] Fig. 17 shows SEQ ID No. 19 to SEQ ID No. 22.

### Description of Embodiments

### (Underlying Knowledge Forming Basis of the Present Disclosure)

As described in the background art, there is a need for a technique for efficiently producing high-quality agricultural crops in a limited arable land. To promote crop production, there is also a need for utilization of a naturally occurring material with a low environmental load in application. In particular, there is a demand for a material that consumes a small amount of fossil energy for the production of the material and has a lower environmental load.

The following techniques has been disclosed as techniques for promoting crop production.

For example, Patent Literature 1 discloses a plant activator that suppresses plant diseases by applying an oxo fatty acid derivative, which is a fatty acid metabolite of a bacterium, to a plant to induce systemic resistance in the plant through the salicylic acid pathway. It has also been reported that since the oxo fatty acid derivative is a naturally occurring fatty acid oxide, the plant activator has a low environmental load and has almost no phytotoxicity to a plant to which the plant activator is applied.

For example, Patent Literature 2 discloses a polypeptide that is composed of part of an elongation factor (EF-Tu) widely conserved in phytopathogenic bacteria and non-phytopathogenic bacteria, is composed of a predetermined amino acid sequence, and has an activity of inducing an immune response of a gramineous plant. It has been reported that the polypeptide is highly safe to human beings, has a very low environmental load, has a relatively low molecular weight, and can therefore be produced easily at a low cost using a bacterium. It has also been reported that the peptide and a plant disease resistance inducing agent containing the peptide are less likely to develop a mutant resistant to the agent and can be applied for extended periods.

For example, Patent Literature 3 discloses the use of a novel bacterium belonging to the genus Paenibacillus with a plant disease controlling action for controlling plant diseases. More specifically, it has been reported that spores, vegetative bacterial cells, dried bacterial cells, whole cultures, lyophilized powders thereof, and the like of a bacterium belonging to the genus Paenibacillus exhibit effects even when applied to plants in various forms.

However, in the related art, a production process of a plant disease resistance inducer by a microorganism, a purification or extraction process of a plant disease resistance inducer, and the like are complicated, take a lot of time and effort, and entail high costs. Furthermore, in the related art described above, purification and extraction of a plant disease resistance inducer causes a loss, such as a decrease in the yield of the plant disease resistance inducer or a decrease in the activity thereof. On the other hand, inoculating a plant with a microorganism itself has different effects depending on the combination of the species of the microorganism used, the species of the target plant, and the properties of soil, thus lacking versatility and having unstable plant disease resistance induction effects. Thus, it is desired to develop a naturally occurring material that can be produced from a less expensive raw material by a simple process and has a high plant disease resistance inducing effect.

The present inventors have focused on a cyanobacterium as a microorganism for use in the production of a plant disease resistance inducer. Cyanobacteria (also called blue-green bacteria or blue-green algae) are a group of eubacteria, photosynthetically split water to produce oxygen, and fix CO₂ in the air by using the obtained energy. Some species of cyanobacteria can also fix nitrogen (N₂) in the air. As described above, cyanobacteria can obtain most of the raw materials (that is, nutrients) and energy required for the growth of the bacterial cells from air, water, and light, and can therefore be cultured with inexpensive raw materials by a simple process.

Cyanobacteria are known to have characteristics of rapid growth and high light-use efficiency and are easier to genetically manipulate than other algae species. Thus, active research and development has been conducted on material production using cyanobacteria among photosynthetic microorganisms. For example, production of fuels, such as ethanol, isobutanol, alkanes, and fatty acids (Patent Literature 4: Japanese Patent No. 6341676) has been reported as an example of material production using cyanobacteria. Furthermore, research and development on the production of materials serving as nutrient sources for organisms has also been conducted. For example, since proteins can be synthesized only by organisms, there is a need for the development of a technique for simply and efficiently producing a protein. As one of biological species used in the technique, cyanobacteria that can utilize light energy and CO₂ in the atmosphere are expected to be utilized, and active research and development thereof has been conducted (Non-Patent Literature 1: Jie Zhou et al., "Discovery of a super-strong promoter enable efficient production of heterologous proteins in cyanobacteria", Scientific Reports, Nature Research, 2014, Vol. 4, Article No. 4500).

For example, the technique described in Non-Patent Literature 1 can achieve efficient expression of a heterologous gene in a cyanobacterium. This technique can be used to produce a desired protein in a cell of a cyanobacterium (hereinafter also referred to as "in a bacterial cell"). However, a protein produced in a cell of a cyanobacterium is rarely secreted from the cell, and it is therefore necessary to disrupt the cell of the cyanobacterium and extract the protein produced in the cell.

In view of the above, the present inventors have found that an outer membrane covering a cell wall of a cyanobacterium can be partially detached from the cell wall to facilitate secretion of a protein and an intracellular metabolite produced in the bacterial cell of the cyanobacterium from the bacterial cell. Furthermore, the present inventors have also found that a secretion product of a cyanobacterium has a plant disease resistance inducing effect. Consequently, a plant disease resistance inducer secreted from a bacterial cell of a cyanobacterium can be efficiently produced without disrupting the bacterial cell. Furthermore, the elimination of extraction or the like makes the biological activity of the plant disease resistance inducer less susceptible to damage. Thus, a plant disease resistance inducing agent containing the secretion product can effectively induce plant disease resistance.

Thus, a plant disease resistance inducing agent and a plant disease resistance inducing method according to the present disclosure can effectively induce plant disease resistance. A method for producing a plant disease resistance inducing agent according to the present disclosure can simply and efficiently produce a plant disease resistance inducing agent.

### Outline of Present Disclosure

An outline of one aspect of the present disclosure is described below.

A plant disease resistance inducing agent according to one aspect of the present disclosure contains a secretion product of a cyanobacterium.

The plant disease resistance inducing agent contains a secretion product involved in the induction of plant disease resistance and can effectively induce resistance to a plant disease.

For example, in a plant disease resistance inducing agent according to one aspect of the present disclosure, the cyanobacterium may be a modified cyanobacterium in which a function of a protein involved in binding between an outer membrane and a cell wall is suppressed or eliminated.

Thus, in the modified cyanobacterium, the binding between the cell wall and the outer membrane (that is, the amount and strength of binding) is partially reduced, and the outer membrane is therefore likely to be partially detached from the cell wall. Thus, in the modified cyanobacterium, a protein and a metabolite produced in a bacterial cell (that is, an intracellular product) are likely to leak out of the outer membrane (that is, out of the bacterial cell). Thus, a protein and a metabolite produced in the bacterial cell of the modified cyanobacterium are easily secreted from the bacterial cell, and therefore, for example, a process of extracting an intracellular product, such as disrupting the bacterial cell, is not required. This is less likely to reduce the biological activity and the yield of the intracellular product and is also less likely to reduce the biological activity and the yield of a substance involved in the induction of plant disease resistance (hereinafter also referred to as a plant disease resistance inducer) among the intracellular products of the modified cyanobacterium. Thus, a plant disease resistance inducing agent according to one aspect of the present disclosure can effectively induce resistance to a plant disease.

For example, in a plant disease resistance inducing agent according to one aspect of the present disclosure, the protein involved in the binding between the outer membrane and the cell wall may be at least one of a surface layer homology (SLH)-domain-containing outer membrane protein and a cell-wall-pyruvate modification enzyme.

In the modified cyanobacterium, for example, (i) this suppresses or eliminates the function of at least one of a SLH-domain-containing outer membrane protein that binds to a cell wall and an enzyme that catalyzes a reaction of modifying a sugar chain bound to the surface of a cell wall with a pyruvate (that is, a cell-wall-pyruvate modification enzyme), or (ii) this represses the expression of at least one of the SLH-domain-containing outer membrane protein and the cell-wall-pyruvate modification enzyme. This reduces the binding (that is, the amount and strength of binding) between the SLH domain of the SLH-domain-containing outer membrane protein in the outer membrane and the covalently linked sugar chain on the surface of the cell wall. Thus, the outer membrane is easily detached from the cell wall at a portion where the binding between the outer membrane and the cell wall is weakened. Consequently, in the modified cyanobacterium, the outer membrane is likely to be partially detached from the cell wall due to a decrease in the binding between the outer membrane and the cell wall, and an intracellular product, such as a protein or a metabolite produced in the bacterial cell, is therefore likely to leak out of the bacterial cell as described above. This improves the secretory productivity of the modified cyanobacterium to secrete a plant disease resistance inducer produced in the bacterial cell from the bacterial cell. Thus, a plant disease resistance inducing agent according to one aspect of the present disclosure contains a plant disease resistance inducer efficiently secreted by the modified cyanobacterium and can therefore effectively induce resistance to a plant disease.

For example, in a plant disease resistance inducing agent according to one aspect of the present disclosure, the SLH-domain-containing outer membrane protein may be Slr1841 consisting of an amino acid sequence of SEQ ID No. 1, NIES970_09470 consisting of an amino acid sequence of SEQ ID No. 2, Anacy _3458 consisting of an amino acid sequence of SEQ ID No. 3, or a protein with an amino acid sequence identity of 50% or more with any of these SLH-domain-containing outer membrane proteins.

In the modified cyanobacterium, for example, (i) this suppresses or eliminates the function of any of the SLH-domain-containing outer membrane proteins represented by SEQ ID Nos. 1 to 3 or a protein with an amino acid sequence identity of 50% or more with any of these SLH-domain-containing outer membrane proteins, or (ii) this represses the expression of any of the SLH-domain-containing outer membrane proteins represented by SEQ ID Nos. 1 to 3 or a protein with an amino acid sequence identity of 50% or more with any of these SLH-domain-containing outer membrane proteins. In the modified cyanobacterium, (i) this suppresses or eliminates the function of the SLH-domain-containing outer membrane protein or a protein with a function equivalent to that of the SLH-domain-containing outer membrane protein in the outer membrane, or (ii) this reduces the expression level of the SLH-domain-containing outer membrane protein or a protein with a function equivalent to that of the SLH-domain-containing outer membrane in the outer membrane. Consequently, in the modified cyanobacterium, a binding domain (for example, SLH domain) for binding of the outer membrane to the cell wall has a reduced amount and strength of binding to the cell wall, and the outer membrane is therefore likely to be partially detached from the cell wall. This promotes the leakage of the intracellular product from the bacterial cell and the leakage of the plant disease resistance inducer produced in the bacterial cell from the bacterial cell. Thus, a plant disease resistance inducing agent according to one aspect of the present disclosure contains the plant disease resistance inducer efficiently secreted by the modified cyanobacterium and can therefore effectively induce plant disease resistance.

For example, in a plant disease resistance inducing agent according to one aspect of the present disclosure, the cell-wall-pyruvate modification enzyme may be Slr0688 consisting of an amino acid sequence of SEQ ID No. 4, Synpcc7942_1529 consisting of an amino acid sequence of SEQ ID No. 5, Anacy_1623 consisting of an amino acid sequence of SEQ ID No. 6, or a protein with an amino acid sequence identity of 50% or more with any of these cell-wall-pyruvate modification enzymes.

In the modified cyanobacterium, for example, (i) this suppresses or eliminates the function of any of the cell-wall-pyruvate modification enzymes represented by SEQ ID Nos. 4 to 6 or a protein with an amino acid sequence identity of 50% or more with any of these cell-wall-pyruvate modification enzymes, or (ii) this represses the expression of any of the cell-wall-pyruvate modification enzymes represented by SEQ ID Nos. 4 to 6 or a protein with an amino acid sequence identity of 50% or more with any of these cell-wall-pyruvate modification enzymes. In the modified cyanobacterium, (i) this suppresses or eliminates the function of the cell-wall-pyruvate modification enzyme or a protein with a function equivalent to that of the enzyme, or (ii) this reduces the expression level of the cell-wall-pyruvate modification enzyme or a protein with a function equivalent to that of the enzyme. This makes it difficult to modify the covalently linked sugar chain on the surface of the cell wall with a pyruvate and therefore reduces the amount and strength of binding of the sugar chain on the cell wall to the SLH domain of the SLH-domain-containing outer membrane protein in the outer membrane. Consequently, in the modified cyanobacterium, the covalently linked sugar chain on the surface of the cell wall is less likely to be modified with a pyruvate, so that the binding strength between the cell wall and the outer membrane is weakened, and the outer membrane is likely to be partially detached from the cell wall. This promotes the leakage of the intracellular product from the bacterial cell and the leakage of the plant disease resistance inducer produced in the bacterial cell from the bacterial cell. Thus, a plant disease resistance inducing agent according to one aspect of the present disclosure contains the plant disease resistance inducer efficiently secreted by the modified cyanobacterium and can therefore effectively induce plant disease resistance.

For example, in a plant disease resistance inducing agent according to one aspect of the present disclosure, the cyanobacterium may be a modified cyanobacterium in which a gene that expresses a protein involved in binding between an outer membrane and a cell wall is deleted or inactivated.

In the modified cyanobacterium, this represses the expression of a protein involved in the binding between the cell wall and the outer membrane or suppresses or eliminates the function of the protein, and the binding (that is, the amount and strength of binding) between the cell wall and the outer membrane is therefore partially reduced. Consequently, in the modified cyanobacterium, the outer membrane is likely to be partially detached from the cell wall, and an intracellular product, such as a protein or a metabolite produced in the bacterial cell, is therefore likely to leak out of the outer membrane, that is, out of the bacterial cell. In the modified cyanobacterium, this improves the secretory productivity of the plant disease resistance inducer produced in the bacterial cell. This eliminates the need for a process of extracting an intracellular product, such as disrupting the bacterial cell, is therefore less likely to reduce the biological activity and the yield of the intracellular product, and is less likely to reduce the biological activity and the yield of the plant disease resistance inducer produced in the bacterial cell. Thus, a plant disease resistance inducing agent according to one aspect of the present disclosure can effectively induce plant disease resistance.

For example, in a plant disease resistance inducing agent according to one aspect of the present disclosure, a gene that expresses the protein involved in the binding between the outer membrane and the cell wall may be at least one of a gene encoding a SLH-domain-containing outer membrane protein and a gene encoding a cell-wall-pyruvate modification enzyme.

In the modified cyanobacterium, this deletes or inactivates at least one of a gene encoding the SLH-domain-containing outer membrane protein and a gene encoding the cell-wall-pyruvate modification enzyme. In the modified cyanobacterium, for example, (i) this represses the expression of at least one of the SLH-domain-containing outer membrane protein and the cell-wall-pyruvate modification enzyme, or (ii) this suppresses or eliminates the function of at least one of the SLH-domain-containing outer membrane protein and the cell-wall-pyruvate modification enzyme. This reduces the binding (that is, the amount and strength of binding) between the SLH domain of the SLH-domain-containing outer membrane protein in the outer membrane and the covalently linked sugar chain on the surface of the cell wall. Thus, the outer membrane is easily detached from the cell wall at a portion where the binding between the outer membrane and the cell wall is weakened. Consequently, in the modified cyanobacterium, the outer membrane is likely to be partially detached from the cell wall due to a decrease in the binding between the outer membrane and the cell wall, a protein and a metabolite produced in the bacterial cell are therefore likely to leak out of the bacterial cell, and a plant disease resistance inducer produced in the bacterial cell is also likely to leak out of the bacterial cell. Thus, a plant disease resistance inducing agent according to one aspect of the present disclosure contains the plant disease resistance inducer efficiently secreted by the modified cyanobacterium and can therefore effectively induce plant disease resistance.

For example, in a plant disease resistance inducing agent according to one aspect of the present disclosure, the gene encoding the SLH-domain-containing outer membrane protein may be slr1841 consisting of a base sequence of SEQ ID No. 7, nies970_09 470 consisting ofa base sequence of SEQ ID No. 8, anacy_3458 consisting of a base sequence of SEQ ID No. 9, or a gene with a base sequence identity of 50% or more with any of these genes.

In the modified cyanobacterium, a gene encoding any of the SLH-domain-containing outer membrane proteins represented by SEQ ID Nos. 7 to 9 or a gene with a base sequence identity of 50% or more with any of these genes is deleted or inactivated. In the modified cyanobacterium, (i) this represses the expression of any of the SLH-domain-containing outer membrane proteins or a protein with a function equivalent to that of any of these proteins, or (ii) this suppresses or eliminates the function of any of the SLH-domain-containing outer membrane proteins or a protein with a function equivalent to that of any of these proteins. Consequently, in the modified cyanobacterium, a binding domain (for example, SLH domain) for binding of the outer membrane to the cell wall has a reduced amount and strength of binding to the cell wall, and the outer membrane is therefore likely to be partially detached from the cell wall. This promotes the leakage of the protein and the metabolite produced in the bacterial cell from the bacterial cell and the leakage of the plant disease resistance inducer produced in the bacterial cell from the bacterial cell. Thus, a plant disease resistance inducing agent according to one aspect of the present disclosure contains the plant disease resistance inducer efficiently secreted by the modified cyanobacterium and can therefore effectively induce plant disease resistance.

For example, in a plant disease resistance inducing agent according to one aspect of the present disclosure, the gene encoding the cell-wall-pyruvate modification enzyme may be slr0688 consisting ofa base sequence of SEQ ID No. 10, synpcc7942_1 529 consisting ofa base sequence of SEQ ID No. 11, anacy_1623 consisting of a base sequence of SEQ ID No. 12, or a gene with a base sequence identity of 50% or more with any of these genes.

In the modified cyanobacterium, this deletes or inactivates a gene encoding any of the cell-wall-pyruvate modification enzymes of SEQ ID Nos. 10 to 12 or a gene with a base sequence identity of 50% or more with the gene encoding any of these enzymes. In the modified cyanobacterium, (i) this represses the expression of any of the cell-wall-pyruvate modification enzymes or a protein with a function equivalent to that of any of these enzymes, or (ii) this suppresses or eliminates the function of any of the cell-wall-pyruvate modification enzymes or a protein with a function equivalent to that of any of these enzymes. This makes it difficult to modify the covalently linked sugar chain on the surface of the cell wall with a pyruvate and therefore reduces the amount and strength of binding of the sugar chain on the cell wall to the SLH domain of the SLH-domain-containing outer membrane protein in the outer membrane. Consequently, in the modified cyanobacterium, a smaller amount of the sugar chain for binding the cell wall to the outer membrane is modified with a pyruvate, so that the binding strength between the cell wall and the outer membrane is weakened, and the outer membrane is likely to be partially detached from the cell wall. This promotes the leakage of the protein and the metabolite produced in the bacterial cell from the bacterial cell and the leakage of the plant disease resistance inducer produced in the bacterial cell from the bacterial cell. Thus, a plant disease resistance inducing agent according to one aspect of the present disclosure contains the plant disease resistance inducer efficiently secreted by the modified cyanobacterium and can therefore effectively induce plant disease resistance.

A plant disease resistance inducing method according to one aspect of the present disclosure uses a plant disease resistance inducing agent containing a secretion product of a cyanobacterium.

A plant disease resistance inducing method according to one aspect of the present disclosure that uses a plant disease resistance inducing agent containing a secretion product involved in the induction of plant disease resistance can effectively induce plant disease resistance.

A method for producing a plant disease resistance inducing agent according to one aspect of the present disclosure includes preparing a cyanobacterium and causing the cyanobacterium to secrete a secretion product involved in the induction of plant disease resistance.

Thus, a secretion product involved in the induction of plant disease resistance produced in a bacterial cell of the cyanobacterium can be secreted only by culturing the cyanobacterium, and a plant disease resistance inducing agent containing the secretion product can therefore be easily and efficiently produced.

Embodiments are more specifically described below with reference to the accompanying drawings.

The following embodiments are general or specific examples. The numerical values, materials, steps, the order of steps, and the like in the following embodiments are only examples and are not intended to limit the present disclosure. Among the constituents in the following embodiments, constituents not described in the independent claims defining the highest level concepts are described as optional constituents.

The accompanying figures are not necessarily precise figures. Like parts are denoted by like reference numerals throughout the figures, and redundant description may be omitted or simplified.

Furthermore, in the following description, a numerical range does not represent only a strict meaning but includes a substantially equivalent range, for example, measuring the amount (for example, the number, the concentration, or the like) of a protein or the range thereof.

Furthermore, in the present description, a bacterial cell and a cell represent one individual of cyanobacteria.

### (Embodiments)

In the present description, the identity of base sequences and amino acid sequences is calculated by the BLAST (Basic Local Alignment Search Tool) algorithm. More specifically, it is calculated by pairwise analysis using the BLAST program available on the website of National Center for Biotechnology Information (NCBI) (https://blast.ncbi.nlm.nih.gov/Blast.cgi). Information on genes of cyanobacteria and proteins encoded by the genes is disclosed in, for example, the NCBI database and Cyanobase (http://genome.microbedb.jp/cyanobase/). The amino acid sequence of a target protein and the base sequence of a gene encoding the protein can be obtained from these databases.

### [1. Plant Disease Resistance Inducing Agent]

First, a plant disease resistance inducing agent according to the present embodiment is described below. The plant disease resistance inducing agent refers to an agent for inducing resistance to a plant disease and controlling the plant disease. The plant disease resistance inducing agent is intended to prevent a disease and may therefore be applied before the development of the disease. The plant disease resistance inducing agent may be used by any of spraying, dusting, immersion, powder coating, application, fumigation, smoking, irrigation, and the like. Specific modes of use include a method of spraying or applying an agent to a plant, a method of immersing a seed of a plant in a liquid containing an agent, a method of spraying an agent to a field where a disease has occurred or a field where a disease is likely to occur, and a method of mixing an agent with soil. The amount of the plant disease resistance inducing agent to be used may be appropriately determined depending on the type of the target plant, the growth stage of the target plant, the properties of soil, the type of dosage form, the application method, the application time, and the like. A plant to which the plant disease resistance inducing agent is to be applied may be any cultivated plant and may be a monocotyledon or a dicotyledon. Examples thereof include, but are not limited to, cruciferous plants, such as cabbage, gramineous plants, such as rice, maize, barley, and wheat, solanaceous plants, such as tomato, eggplant, potato, and tobacco, cucurbitaceous plants, such as cucumber, melon, and pumpkin, leguminous plants, such as soybean, pea, common bean, alfalfa, and peanut, rosaceous plants, such as strawberry, apple, and pear, moraceous plants, such as mulberry, malvaceous plants, such as cotton, umbelliferous plants, such as carrot, parsley, and celery, Compositae plants, such as burdock and lettuce, and Vitaceae plants, such as grape.

A plant disease resistance inducing agent contains a secretion product of a cyanobacterium. The cyanobacterium is, for example, a modified cyanobacterium in which the function of a protein involved in binding between an outer membrane and a cell wall (hereinafter also referred to as a binding-related protein) in a cyanobacterium (hereinafter also referred to as a parent cyanobacterium) is suppressed or eliminated. The cyanobacterium (that is, parent cyanobacterium) and the modified cyanobacterium are described later.

The plant disease resistance inducing agent contains a secretion product involved in the induction of plant disease resistance secreted by the cyanobacterium. The phrase "involved in the induction of plant disease resistance" may include not only being directly involved in the induction of plant disease resistance but also being indirectly (in other words, secondarily) involved in the induction of plant disease resistance. Thus, the plant disease resistance inducing agent can effectively induce resistance to a plant disease and can be applied to a plant to improve the yield and quality of the plant.

The secretion product includes a protein and a metabolite produced in a bacterial cell of the cyanobacterium (hereinafter also referred to as an intracellular product). The intracellular product includes a substance that induces resistance to a plant disease (so-called plant disease resistance inducer).

The intracellular product is, for example, an organic matter degrading enzyme, such as peptidase, nuclease, or phosphatase, a DNA metabolism-related substance, such as adenosine or guanosine, an intracellular molecule involved in the promotion of a nucleic acid (for example, DNA or RNA) synthesis, such as p-aminobenzoic acid or spermidine, a ketone body, such as 3-hydroxybutyric acid, or an organic acid, such as gluconic acid. The secretion product of the cyanobacterium may be a mixture of these intracellular products.

### [2. Method for Producing Plant Disease Resistance Inducing Agent]

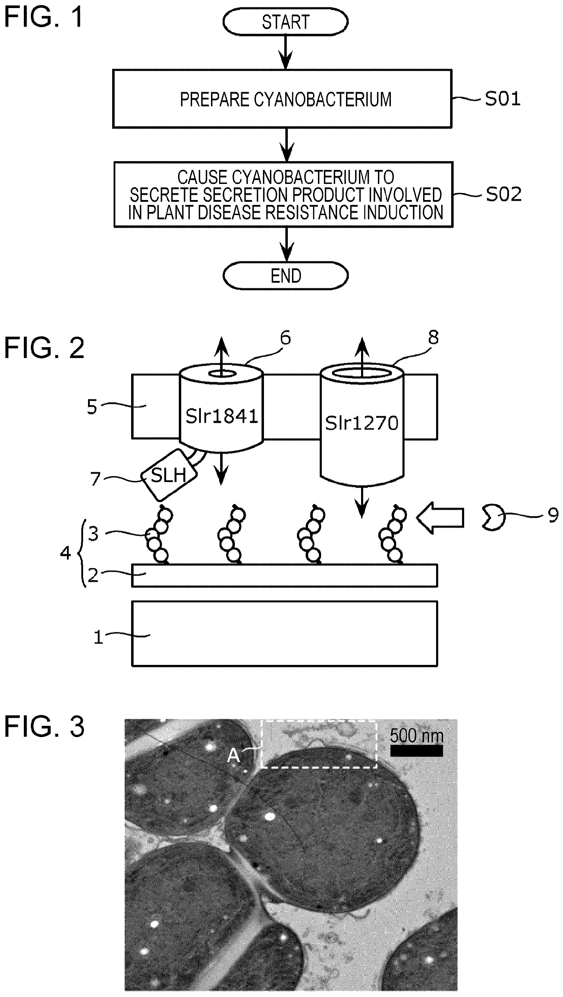

Next, a method for producing a plant disease resistance inducing agent according to the present embodiment is described with reference to Fig. 1. Fig. 1 is a flow chart of an example of the method for producing a plant disease resistance inducing agent according to the present embodiment.

The method for producing a plant disease resistance inducing agent according to the present embodiment includes a step of preparing a cyanobacterium (step S01) and a step of causing the cyanobacterium to secrete a secretion product involved in plant disease resistance induction (step S02). As described above, the cyanobacterium is, for example, a modified cyanobacterium in which the function of a protein involved in binding between an outer membrane and a cell wall in a cyanobacterium (so-called parent cyanobacterium) is suppressed or eliminated. Thus, in the step S01, a modified cyanobacterium may be prepared. In such a case, in the step S02, the modified cyanobacterium secretes a secretion product involved in plant disease resistance induction. As described above, the secretion product includes a protein and a metabolite produced in a bacterial cell of the cyanobacterium (that is, an intracellular product). These intracellular products include a substance involved in the induction of plant disease resistance (that is, a plant disease resistance inducer).

Each step is more specifically described below.

In the step S01, the cyanobacterium is prepared. The phrase "preparing a cyanobacterium" means that the state of the cyanobacterium is adjusted so that the cyanobacterium can secrete a secretion product and is, for example, to reconstruct a bacterial cell from a lyophilized product or a glycerol stock of the cyanobacterium or to recover the cyanobacterium that has finished secreting a plant disease resistance material in the step S02. When the cyanobacterium is a modified cyanobacterium, preparing the cyanobacterium is, for example, to genetically modify a parent cyanobacterium to prepare the modified cyanobacterium, to reconstruct a bacterial cell from a lyophilized product or a glycerol stock of the modified cyanobacterium, or to recover the modified cyanobacterium that has finished secreting a plant disease resistance inducer in the step S02.

In the step S02, the cyanobacterium is caused to secrete a secretion product involved in the induction of plant disease resistance. The cyanobacterium in the present embodiment is a modified cyanobacterium in which the function of a protein involved in binding between an outer membrane and a cell wall in the cyanobacterium (that is, parent cyanobacterium) is suppressed or eliminated, and a protein and a metabolite produced in the bacterial cell are therefore easily secreted from the outer membrane (that is, from the bacterial cell). These intracellular products include a substance involved in the induction of plant disease resistance. Thus, in the step S02, the modified cyanobacterium is cultured under predetermined conditions, and an intracellular product involved in the induction of plant disease resistance is secreted from a bacterial cell.

The cyanobacterium can typically be cultured based on liquid culture using a BG-11 culture medium (see Table 2) or based on a modification thereof. Thus, the modified cyanobacterium may be cultured in the same manner. The culture period of the cyanobacterium for producing a plant disease resistance inducing agent may be a period during which a protein and a metabolite can be accumulated in a high concentration under the conditions that a bacterial cell is sufficiently grown, and is, for example, in the range of 1 to 3 days or 4 to 7 days. The culture method is, for example, aeration agitation culture or shake culture.

When cultured under the conditions described above, the modified cyanobacterium produces a protein and a metabolite in a bacterial cell (that is, an intracellular product) and secretes the intracellular product into a culture fluid. The intracellular product includes an intracellular product involved in the induction of plant disease resistance (that is, a plant disease resistance inducer). When the intracellular product secreted into the culture fluid is recovered, the culture fluid may be filtered, centrifuged, or the like to remove solid components, such as cells (that is, bacterial cells), from the culture fluid to recover a culture supernatant. In the method for producing a plant disease resistance inducing agent according to the present embodiment, a secretion product containing an intracellular product involved in the induction of plant disease resistance (that is, a plant disease resistance inducer) is secreted from a cell of the modified cyanobacterium, and it is therefore not necessary to disrupt the cell to recover the plant disease resistance inducer. Thus, the modified cyanobacterium remained after the recovery of the plant disease resistance inducer can be repeatedly used to produce a plant disease resistance inducing agent.

The method for recovering the plant disease resistance inducer secreted into the culture fluid is not limited to the example described above, and the plant disease resistance inducer in the culture fluid may be recovered while culturing the modified cyanobacterium. For example, a protein-permeable membrane may be used to recover the plant disease resistance inducer that has permeated through the permeable membrane. Thus, the plant disease resistance inducer in the culture fluid can be recovered while culturing the modified cyanobacterium, and it is therefore not necessary to remove the bacterial cells of the modified cyanobacterium from the culture fluid. Thus, the plant disease resistance inducing agent can be more simply and efficiently produced.

Furthermore, the elimination of the recovery treatment of bacterial cells from the culture fluid and the disruption treatment of the bacterial cells can reduce the damage and stress to the modified cyanobacterium. Thus, the secretory productivity of the plant disease resistance inducer of the modified cyanobacterium is less likely to decrease, and the modified cyanobacterium can be used for a longer period.

As described above, the modified cyanobacterium of the present embodiment can be used to simply and efficiently produce a plant disease resistance inducing agent.

The cyanobacterium (so-called parent cyanobacterium) and the modified cyanobacterium are described below. Hereinafter, the parent cyanobacterium is referred to as a cyanobacterium, and the cyanobacterium in the present embodiment is referred to as a modified cyanobacterium.

### [3. Cyanobacterium]

Cyanobacteria are also called blue-green algae or blue-green bacteria and are a group of prokaryotes that collect light energy with chlorophyll and electrolyze water with the obtained energy to produce oxygen and perform photosynthesis. There is a variety of cyanobacteria. For example, in terms of the cell shape, there are unicellular species, such as Synechocystis sp. PCC 6803, and filamentous species, such as Anabaena sp. PCC 7120, in which multiple cells are connected. In terms of the growth environment, there are thermophilic species, such as Thermosynechococcus elongatus, marine species, such as Synechococcus elongatus, and freshwater species, such as Synechocystis. There are also many species with unique characteristics, such as Microcystis aeruginosa, which has gas vesicles and produces toxins, and Gloeobacter violaceus, which does not have thylakoids and has a protein called phycobilisome, which is a light-harvesting antenna, in the plasma membrane.

Fig. 2 is a view schematically illustrating a cell surface layer of a cyanobacterium. As illustrated in Fig. 2, the cell surface layer of the cyanobacterium is composed of, in order from the inside, a plasma membrane (also referred to as an inner membrane 1), peptidoglycan 2, and an outer membrane 5, which is a lipid membrane forming the outermost layer of the cell. A sugar chain 3 composed of glucosamine, mannosamine, and the like is covalently bound to the peptidoglycan 2, and a pyruvate is bound to a covalently linked sugar chain 3 (Non-Patent Literature 2: Jurgens and Weckesser, 1986, J. Bacteriol., 168: 568-573). In the present description, the peptidoglycan 2 and the covalently linked sugar chain 3 are collectively referred to as a cell wall 4. The space between the plasma membrane (that is, the inner membrane 1) and the outer membrane 5 is called the periplasm, in which various enzymes are present that are involved in the degradation of a protein or the formation of a three-dimensional structure, the degradation of a lipid or a nucleic acid, the uptake of an extracellular nutrient, or the like.

The SLH-domain-containing outer membrane protein (for example, Slr1841 in the drawing) is composed of a C-terminal domain embedded in the lipid membrane (also referred to as the outer membrane 5) and an N-terminal SLH domain 7 protruding from the lipid membrane and is widely distributed in a cyanobacterium and a bacterium belonging to the class Negativicutes, which is a group of Gram-negative bacteria (Non-Patent Literature 3: Kojima et al., 2016, Biosci. Biotech. Biochem., 10: 1954-1959). The domain embedded in the lipid membrane (that is, the outer membrane 5) forms a channel to allow permeation of a hydrophilic substance through the outer membrane, and the SLH domain 7 functions to bind to the cell wall 4 (Non-Patent Literature 4: Kowata et al., 2017, J. Bacteriol., 199: e00371-17). In order for the SLH domain 7 to bind to the cell wall 4, the covalently linked sugar chain 3 in the peptidoglycan 2 needs to be modified with a pyruvate (Non-Patent Literature 5: Kojima et al., 2016, J. Biol. Chem., 291: 20198-20209). A gene encoding a SLH-domain-containing outer membrane protein 6 is, for example, slr1841 or slr1908 carried by Synechocystis sp. PCC 6803 or oprB carried by Anabaena sp. 90.

An enzyme that catalyzes a pyruvate modification reaction of the covalently linked sugar chain 3 in the peptidoglycan 2 (hereinafter referred to as a cell-wall-pyruvate modification enzyme 9) has been identified in Bacillus anthracis, which is a Gram-positive bacterium and named CsaB (Non-Patent Literature 6: Mesnage et al., 2000, EMBO J., 19: 4473-4484). In cyanobacteria with published genome base sequences, many species have a gene encoding a homologous protein with an amino acid sequence identity of 30% or more with CsaB. Examples thereof include slr0688 carried by Synechocystis sp. PCC 6803 and syn7502_03092 carried by Synechococcus sp. 7502.

In cyanobacteria, photosynthetically fixed CO₂ is converted to precursors of various amino acids and intracellular molecules through multistep enzymatic reactions. Using them as raw materials, proteins and metabolites are synthesized in the cytoplasm of cyanobacteria. Some of these proteins and metabolites function in the cytoplasm, and others are transported from the cytoplasm to the periplasm and function in the periplasm. However, cases in which proteins and metabolites are actively secreted from cells have not been reported in cyanobacteria.

Cyanobacteria have high photosynthetic abilities and do not necessarily need to take in organic substances from the outside as nutrients. Thus, cyanobacteria have very few organic permeable channel proteins in the outer membrane 5, such as an organic channel protein 8 (for example, Slr1270) of Fig. 2. For example, in Synechocystis sp. PCC 6803, the organic channel protein 8 that allows an organic substance to permeate is only approximately 4% of the total protein of the outer membrane 5. On the other hand, to efficiently take inorganic ions necessary for growth into cells, cyanobacteria have many ion channel proteins in the outer membrane 5 that allow only inorganic ions to permeate, such as the SLH-domain-containing outer membrane protein 6 (for example, Slr1841) of Fig. 2. For example, in Synechocystis sp. PCC 6803, ion channel proteins that allow inorganic ions to permeate account for approximately 80% of the total protein of the outer membrane 5.

As described above, since cyanobacteria have very few channels that are permeable to organic substances, such as proteins, in the outer membrane 5, it is considered to be difficult to actively secrete proteins and metabolites produced in bacterial cells from the bacterial cell.

### [4. Modified Cyanobacterium]

Next, the modified cyanobacterium is described with reference to Fig. 2.

The cyanobacterium in the present embodiment is the modified cyanobacterium in which the function of a protein involved in the binding between the outer membrane 5 and the cell wall 4 (so-called binding-related protein) in the cyanobacterium (so-called parent cyanobacterium) is suppressed or eliminated. More specifically, for example, in the modified cyanobacterium, the total amount of the protein involved in the binding between the outer membrane 5 and the cell wall 4 in the cyanobacterium (that is, the binding-related protein) is reduced to 30% or more and 70% or less of the total amount of the protein in the parent strain (that is, the parent cyanobacterium). For example, the phrase "the total amount of the binding-related protein is reduced to 30% of the total amount of the protein in the parent strain" means that 70% of the total amount of the protein in the parent strain is lost and 30% of the total amount remains. Thus, in the modified cyanobacterium, the binding (for example, the amount and strength of binding) between the outer membrane 5 and the cell wall 4 is partially reduced, and the outer membrane 5 is therefore likely to be partially detached from the cell wall 4. Thus, the modified cyanobacterium has improved secretory productivity of an intracellular product, such as a protein or a metabolite produced in a bacterial cell, secreted from the bacterial cell. As described above, the intracellular product includes an intracellular product involved in the induction of plant disease resistance (that is, a plant disease resistance inducer). Thus, the modified cyanobacterium also has improved secretory productivity of the plant disease resistance inducer to secrete a plant disease resistance inducer produced in the bacterial cell from the bacterial cell. Furthermore, since it is not necessary to recover the plant disease resistance inducer by disrupting the bacterial cell, the modified cyanobacterium can be repeatedly used even after the plant disease resistance inducer is recovered. **In** the present description, the production of a protein and a metabolite in a bacterial cell by a modified cyanobacterium is referred to as production, and the secretion of the produced protein and metabolite from the bacterial cell is referred to as secretory production.

The protein involved in the binding between the outer membrane 5 and the cell wall 4 may be, for example, at least one of the SLH-domain-containing outer membrane protein 6 and the cell-wall-pyruvate modification enzyme 9. **In** the present embodiment, in the modified cyanobacterium, for example, the function of at least one of the SLH-domain-containing outer membrane protein 6 and the cell-wall-pyruvate modification enzyme 9 is suppressed or eliminated. For example, in the modified cyanobacterium, (i) the function of at least one of the SLH-domain-containing outer membrane protein 6 and the cell-wall-pyruvate modification enzyme 9 may be suppressed or eliminated, and (ii) at least one of the expression of the SLH-domain-containing outer membrane protein 6 that binds to the cell wall 4 and the expression of an enzyme that catalyzes a pyruvate modification reaction of a sugar chain bound to the surface of the cell wall 4 (that is, the cell-wall-pyruvate modification enzyme 9) may be repressed. This reduces the binding (that is, the amount and strength of binding) between the SLH domain 7 of the SLH-domain-containing outer membrane protein 6 in the outer membrane 5 and the covalently linked sugar chain 3 on the surface of the cell wall 4. Thus, the outer membrane 5 is easily detached from the cell wall 4 at a portion where these bonds are weakened. When the outer membrane 5 is partially detached from the cell wall 4, an intracellular product, such as a protein or a metabolite, present in a cell of the modified cyanobacterium, particularly in the periplasm, is likely to leak out of the cell (out of the outer membrane 5). This improves the secretory productivity of the modified cyanobacterium to secrete a plant disease resistance inducer produced in the bacterial cell from the bacterial cell.

A cyanobacterium modified so that the outer membrane 5 is partially detached from the cell wall 4 by suppressing the function of at least one binding-related protein of the SLH-domain-containing outer membrane protein 6 and the cell-wall-pyruvate modification enzyme 9 is more specifically described.

The type of cyanobacterium before repression or loss of at least one of the expression of the SLH-domain-containing outer membrane protein 6 and the expression of the cell-wall-pyruvate modification enzyme 9 (that is, parent cyanobacterium), which serves as a parent microorganism of the modified cyanobacterium in the present embodiment, is not particularly limited and may be any type. For example, the parent cyanobacterium may be in the genus Synechocystis, Synechococcus, Anabaena, or Thermosynechococcus, particularly Synechocystis sp. PCC 6803, Synechococcus sp. PCC 7942, or Thermosynechococcus elongatus BP-1.

The amino acid sequences of the SLH-domain-containing outer membrane protein 6 and the enzyme catalyzing the cell wall-pyruvate modification reaction (that is, the cell-wall-pyruvate modification enzyme 9) in these parent cyanobacteria, the base sequences of genes encoding their binding-related proteins, and the positions of the genes in the chromosomal DNA or plasmid can be found in the NCBI database and Cyanobase.

The SLH-domain-containing outer membrane protein 6 and the cell-wall-pyruvate modification enzyme 9 with a suppressed or eliminated function in the modified cyanobacterium according to the present embodiment may be those of any parent cyanobacterium that has the SLH-domain-containing outer membrane protein 6 and the cell-wall-pyruvate modification enzyme 9, and are not limited by the location of genes encoding them (for example, in the chromosomal DNA or plasmid).

For example, the SLH-domain-containing outer membrane protein 6 may be Slr1841, Slr1908, Slr0042, or the like when the parent cyanobacterium is in the genus Synechocystis, may be NIES970_09470 or the like when the parent cyanobacterium is in the genus Synechococcus, may be Anacy_5815, Anacy _3458, or the like when the parent cyanobacterium is in the genus Anabaena, may be A0A0F6U6F8_MICAE or the like when the parent cyanobacterium is in the genus Microcystis, may be A0A3B8XX12_9CYAN or the like when the parent cyanobacterium is in the genus Cyanothece, may be AOA1Q8ZE23_9CYAN or the like when the parent cyanobacterium is in the genus Leptolyngbya, may be A0A1Z4R6U0_9CYAN when the parent cyanobacterium is in the genus Calothrix, may be A0A1C0VG86_9NOSO or the like when the parent cyanobacterium is in the genus Nostoc, may be B1WRN6_CROS5 or the like when the parent cyanobacterium is in the genus Crocosphaera, or may be K9TAE4_9CYAN or the like when the parent cyanobacterium is in the genus Pleurocapsa.

More specifically, the SLH-domain-containing outer membrane protein 6 may be, for example, Slr1841 of Synechocystis sp. PCC 6803 (SEQ ID No. 1), NIES970_09470 of Synechococcus sp. NIES-970 (SEQ ID No. 2), or Anacy_3458 of Anabaena cylindrica PCC 7122 (SEQ ID No. 3). It may be a protein with an amino acid sequence identity of 50% or more with the SLH-domain-containing outer membrane protein 6.

In the modified cyanobacterium, for example, (i) this may suppress or eliminate the function of any of the SLH-domain-containing outer membrane proteins 6 represented by SEQ ID Nos. 1 to 3 or a protein with an amino acid sequence identity of 50% or more with any of these SLH-domain-containing outer membrane proteins 6, or (ii) this may repress the expression of any of the SLH-domain-containing outer membrane proteins 6 represented by SEQ ID Nos. 1 to 3 or a protein with an amino acid sequence identity of 50% or more with any of these SLH-domain-containing outer membrane proteins 6. Thus, in the modified cyanobacterium, (i) the function of the SLH-domain-containing outer membrane protein 6 or a protein with a function equivalent to that of the SLH-domain-containing outer membrane protein 6 in the outer membrane 5 is suppressed or eliminated, or (ii) the expression level of the SLH-domain-containing outer membrane protein 6 or a protein with a function equivalent to that of the SLH-domain-containing outer membrane protein 6 in the outer membrane 5 is reduced. In the modified cyanobacterium, this reduces the amount and strength of binding between the cell wall 4 and the binding domain (for example, the SLH domain 7) for binding the outer membrane 5 to the cell wall 4, and the outer membrane 5 is therefore likely to be partially detached from the cell wall 4. In the modified cyanobacterium, this promotes the leakage of the intracellular product from the bacterial cell and the leakage of the plant disease resistance inducer produced in the bacterial cell from the bacterial cell.

In general, it is said that proteins with an amino acid sequence identity of 30% or more have highly homologous three-dimensional structures and are likely to have an equivalent function. Thus, the SLH-domain-containing outer membrane protein 6 with a suppressed or eliminated function may be, for example, a protein or polypeptide having an amino acid sequence identity of 40% or more, preferably 50% or more, more preferably 60% or more, still more preferably 70% or more, still more preferably 80% or more, still more preferably 90% or more, with the amino acid sequence of any of the SLH-domain-containing outer membrane proteins 6 represented by SEQ ID Nos. 1 to 3, and having a function of binding to the covalently linked sugar chain 3 of the cell wall 4.

For example, the cell-wall-pyruvate modification enzyme 9 may be Slr0688 or the like when the parent cyanobacterium is in the genus Synechocystis, may be Syn7502_03092, Synpcc7942_1529, or the like when the parent cyanobacterium is in the genus Synechococcus, may be ANA_C20348, Anacy_1623, or the like when the parent cyanobacterium is in the genus Anabaena, may be CsaB (NCBI Accession ID: TRU80220) or the like when the parent cyanobacterium is in the genus Microcystis, may be CsaB (NCBI Accession ID: WP_107667006.1) when the parent cyanobacterium is in the genus Cyanothece, may be CsaB (NCBI Accession ID: WP_026079530.1) or the like when the parent cyanobacterium is in the genus Spirulina, may be CsaB (NCBI Accession ID: WP_096658142.1) or the like when the parent cyanobacterium is in the genus Calothrix, may be CsaB (NCBI Accession ID: WP_099068528.1) or the like when the parent cyanobacterium is in the genus Nostoc, may be CsaB (NCBI Accession ID: WP_012361697.1) or the like when the parent cyanobacterium is in the genus Crocosphaera, and may be CsaB (NCBI Accession ID: WP_036798735) or the like when the parent cyanobacterium is in the genus Pleurocapsa.

More specifically, the cell-wall-pyruvate modification enzyme 9 may be, for example, Slr0688 of Synechocystis sp. PCC 6803 (SEQ ID No. 4), Synpcc7942_1529 of Synechococcus sp. PCC 7942 (SEQ ID No. 5), or Anacy_1623 of Anabaena cylindrica PCC 7122 (SEQ ID No. 6). It may also be a protein with an amino acid sequence identity of 50% or more with the cell-wall-pyruvate modification enzyme 9.

Thus, in the modified cyanobacterium, for example, (i) the function of any of the cell-wall-pyruvate modification enzymes 9 represented by SEQ ID Nos. 4 to 6 or a protein with an amino acid sequence identity of 50% or more with any of these cell-wall-pyruvate modification enzymes 9 may be suppressed or eliminated, and (ii) the expression of any of the cell-wall-pyruvate modification enzymes 9 represented by SEQ ID Nos. 4 to 6 or a protein with an amino acid sequence identity of 50% or more with any of these cell-wall-pyruvate modification enzymes 9 may be repressed. In the modified cyanobacterium, (i) this suppresses or eliminates the function of the cell-wall-pyruvate modification enzyme 9 or a protein with a function equivalent to that of the enzyme, or (ii) this reduces the expression level of the cell-wall-pyruvate modification enzyme 9 or a protein with a function equivalent to that of the enzyme. This makes it difficult to modify the covalently linked sugar chain 3 on the surface of the cell wall 4 with a pyruvate and reduces the amount and strength of binding of the sugar chain 3 of the cell wall 4 to the SLH domain 7 of the SLH-domain-containing outer membrane protein 6 in the outer membrane 5. Thus, in the modified cyanobacterium according to the present embodiment, the covalently linked sugar chain 3 on the surface of the cell wall 4 is less likely to be modified with a pyruvate, so that the binding strength between the cell wall 4 and the outer membrane 5 is weakened, and the outer membrane 5 is likely to be partially detached from the cell wall 4. In the modified cyanobacterium, this promotes the leakage of the intracellular product from the bacterial cell and the leakage of the plant disease resistance inducer produced in the bacterial cell from the bacterial cell.

Furthermore, as described above, it is said that proteins with an amino acid sequence identity of 30% or more are likely to have an equivalent function. Thus, the cell-wall-pyruvate modification enzyme 9 with a suppressed or eliminated function may be, for example, a protein or polypeptide having an amino acid sequence identity of 40% or more, preferably 50% or more, more preferably 60% or more, still more preferably 70% or more, still more preferably 80% or more, still more preferably 90% or more, with the amino acid sequence of any of the cell-wall-pyruvate modification enzymes 9 represented by SEQ ID Nos. 4 to 6, and having a function of catalyzing the reaction of modifying the covalently linked sugar chain 3 of the peptidoglycan 2 of the cell wall 4 with a pyruvate.

In the present description, the suppression or elimination of the function of the SLH-domain-containing outer membrane protein 6 means the suppression or elimination of the ability of the protein to bind to the cell wall 4, the suppression or elimination of the transport of the protein to the outer membrane 5, or the suppression or elimination of the ability of the protein to be embedded in the outer membrane 5 and function.

The suppression or elimination of the function of the cell-wall-pyruvate modification enzyme 9 means that the protein suppresses or eliminates the function of modifying the covalently linked sugar chain 3 of the cell wall 4 with a pyruvate.

The means for suppressing or eliminating the function of these proteins may be any means commonly used to suppress or eliminate the function of proteins. The means may be, for example, to delete or inactivate a gene encoding the SLH-domain-containing outer membrane protein 6 and a gene encoding the cell-wall-pyruvate modification enzyme 9, to inhibit the transcription of these genes, to inhibit the translation of transcription products of these genes, or to administer an inhibitor that specifically inhibits these proteins.

In the present embodiment, a gene that expresses a protein involved in the binding between the outer membrane 5 and the cell wall 4 is deleted or inactivated in the modified cyanobacterium. In the modified cyanobacterium, this represses the expression of a protein involved in the binding between the cell wall 4 and the outer membrane 5 or suppresses or eliminates the function of the protein, and therefore partially reduces the binding (that is, the amount and strength of binding) between the cell wall 4 and the outer membrane 5. Consequently, in the modified cyanobacterium, the outer membrane 5 is likely to be partially detached from the cell wall 4, and an intracellular product, such as a protein or a metabolite produced in the bacterial cell, is therefore likely to leak out of the outer membrane 5, that is, out of the bacterial cell. Thus, the modified cyanobacterium has improved secretory productivity of the plant disease resistance inducer to secrete a plant disease resistance inducer produced in the bacterial cell from the bacterial cell. This eliminates the need for a process of extracting an intracellular product, such as disrupting the bacterial cell, and is therefore less likely to reduce the biological activity and the yield of the intracellular product. This is less likely to reduce the biological activity and the yield of a plant disease resistance inducer produced in a bacterial cell, and a plant disease resistance inducing agent with an improved plant disease resistance inducing effect can therefore be produced. Furthermore, since the process of extracting an intracellular product is not required, the modified cyanobacterium can be repeatedly used to produce the plant disease resistance inducer even after the plant disease resistance inducer is recovered.

The gene that expresses a protein involved in the binding between the outer membrane 5 and the cell wall 4 may be, for example, at least one of a gene encoding the SLH-domain-containing outer membrane protein 6 and a gene encoding the cell-wall-pyruvate modification enzyme 9. In the modified cyanobacterium, at least one of a gene encoding the SLH-domain-containing outer membrane protein 6 and a gene encoding the cell-wall-pyruvate modification enzyme 9 is deleted or inactivated. In the modified cyanobacterium, for example, (i) this represses the expression of at least one of the SLH-domain-containing outer membrane protein 6 and the cell-wall-pyruvate modification enzyme 9, or (ii) this suppresses or eliminates the function of at least one of the SLH-domain-containing outer membrane protein 6 and the cell-wall-pyruvate modification enzyme 9. This reduces the binding (that is, the amount and strength of binding) between the SLH domain 7 of the SLH-domain-containing outer membrane protein 6 in the outer membrane 5 and the covalently linked sugar chain 3 on the surface of the cell wall 4. Thus, the outer membrane 5 is easily detached from the cell wall 4 at a portion where the binding between the outer membrane 5 and the cell wall 4 is weakened. Consequently, in the modified cyanobacterium, the outer membrane 5 is likely to be partially detached from the cell wall 4 due to a decrease in the binding between the outer membrane 5 and the cell wall 4, and a protein and a metabolite produced in the bacterial cell are therefore likely to leak out of the bacterial cell. Thus, the plant disease resistance inducer produced in the bacterial cell of the modified cyanobacterium is also likely to leak out of the bacterial cell.

In the present embodiment, to suppress or eliminate the function of at least one of the SLH-domain-containing outer membrane protein 6 and the cell-wall-pyruvate modification enzyme 9 in a cyanobacterium, for example, transcription of at least one of a gene encoding the SLH-domain-containing outer membrane protein 6 and a gene encoding the cell-wall-pyruvate modification enzyme 9 may be repressed.

For example, the gene encoding the SLH-domain-containing outer membrane protein 6 may be slr1841, slr1908, slr0042, or the like when the parent cyanobacterium is in the genus Synechocystis, may be nies970_09470 or the like when the parent cyanobacterium is in the genus Synechococcus, may be anacy_5815, anacy_3458, or the like when the parent cyanobacterium is in the genus Anabaena, may be A0A0F6U6F8_MICAE or the like when the parent cyanobacterium is in the genus Microcystis, may be A0A3B8XX12_9CYAN or the like when the parent cyanobacterium is in the genus Cyanothece, may be A0A1Q8ZE23_9CYAN or the like when the parent cyanobacterium is in the genus Leptolyngbya, may be A0A1Z4R6U0_9CYAN or the like when the parent cyanobacterium is in the genus Calothrix, may be A0A1C0VG86_9NOSO or the like when the parent cyanobacterium is in the genus Nostoc, may be B1WRN6_CROS5 or the like when the parent cyanobacterium is in the genus Crocosphaera, and may be K9TAE4_9CYAN or the like when the parent cyanobacterium is in the genus Pleurocapsa. The base sequences of these genes are available from the NCBI database or Cyanobase.

More specifically, the gene encoding the SLH-domain-containing outer membrane protein 6 may be slr1841 of Synechocystis sp. PCC 6803 (SEQ ID No. 7), nies970_09470 of Synechococcus sp. NIES-970 (SEQ ID No. 8), anacy_3458 of Anabaena cylindrica PCC 7122 (SEQ ID No. 9), or a gene with an amino acid sequence identity of 50% or more with these genes.

In the modified cyanobacterium, this deletes or inactivates a gene encoding any of the SLH-domain-containing outer membrane proteins 6 represented by SEQ ID Nos. 7 to 9 or a gene with a base sequence identity of 50% or more with any of these genes. In the modified cyanobacterium, (i) this represses the expression of any of the SLH-domain-containing outer membrane proteins 6 or a protein with a function equivalent to that of any of these proteins, or (ii) this suppresses or eliminates the function of any of the SLH-domain-containing outer membrane proteins 6 or a protein with a function equivalent to that of any of these proteins. In the modified cyanobacterium, this reduces the amount and strength of binding between the cell wall 4 and the binding domain (for example, the SLH domain 7) for binding the outer membrane 5 to the cell wall 4, and the outer membrane 5 is therefore likely to be partially detached from the cell wall 4. This promotes the leakage of the protein and the metabolite produced in the bacterial cell from the bacterial cell and the leakage of the plant disease resistance inducer produced in the bacterial cell from the bacterial cell.

As described above, it is said that proteins with an amino acid sequence identity of 30% or more are likely to have an equivalent function. Thus, it is thought that a gene encoding a protein with a base sequence identity of 30% or more is likely to express a protein with a function equivalent to that of the protein. Thus, the gene encoding the SLH-domain-containing outer membrane protein 6 with a suppressed or eliminated function may be, for example, a gene that has a base sequence identity of 40% or more, preferably 50% or more, more preferably 60% or more, still more preferably 70% or more, still more preferably 80% or more, still more preferably 90% or more, with the base sequence of any of genes encoding the SLH-domain-containing outer membrane proteins 6 represented by SEQ ID Nos. 7 to 9, and that encodes a protein or polypeptide with a function of binding to the covalently linked sugar chain 3 of the cell wall 4.

For example, the gene encoding the cell-wall-pyruvate modification enzyme 9 may be slr0688 or the like when the parent cyanobacterium is in the genus Synechocystis, may be syn7502_03092, synpcc7942_1529, or the like when the parent cyanobacterium is in the genus Synechococcus, may be ana_C20348, anacy_1623, or the like when the parent cyanobacterium is in the genus Anabaena, may be csaB (NCBI Accession ID: TRU80220) or the like when the parent cyanobacterium is in the genus Microcystis, may be csaB (NCBI Accession ID: WP_107667006.1) when the parent cyanobacterium is in the genus Cyanothece, may be csaB (NCBI Accession ID: WP_026079530.1) or the like when the parent cyanobacterium is in the genus Spirulina, may be csaB (NCBI Accession ID: WP_096658142.1) or the like when the parent cyanobacterium is in the genus Calothrix, may be csaB (NCBI Accession ID: WP_099068528.1) or the like when the parent cyanobacterium is in the genus Nostoc, may be csaB (NCBI Accession ID: WP_012361697.1) or the like when the parent cyanobacterium is in the genus Crocosphaera, and may be csaB (NCBI Accession ID: WP_036798735) or the like when the parent cyanobacterium is in the genus Pleurocapsa. The base sequences of these genes are available from the NCBI database or Cyanobase.

More specifically, the gene encoding the cell-wall-pyruvate modification enzyme 9 may be, for example, slr0688 of Synechocystis sp. PCC 6803 (SEQ ID No. 10), synpcc7942_1529 of Synechococcus sp. PCC 7942 (SEQ ID No. 11), or anacy_1623 of Anabaena cylindrica PCC 7122 (SEQ ID No. 12). It may also be a gene with a base sequence identity of 50% or more with these genes.

In the modified cyanobacterium, this deletes or inactivates a gene encoding any of the cell-wall-pyruvate modification enzymes 9 represented by SEQ ID Nos. 10 to 12 or a gene with a base sequence identity of 50% or more with a gene encoding any of these enzymes. In the modified cyanobacterium, (i) this represses the expression of any of the cell-wall-pyruvate modification enzymes 9 or a protein with a function equivalent to that of any of these enzymes, or (ii) this suppresses or eliminates the function of any of the cell-wall-pyruvate modification enzymes 9 or a protein with a function equivalent to that of any of these enzymes. This makes it difficult to modify the covalently linked sugar chain 3 on the surface of the cell wall 4 with a pyruvate and reduces the amount and strength of binding of the sugar chain 3 of the cell wall 4 to the SLH domain 7 of the SLH-domain-containing outer membrane protein 6 in the outer membrane 5. Thus, in the modified cyanobacterium according to the present embodiment, the amount of modification of the sugar chain 3 for binding of the cell wall 4 to the outer membrane 5 with a pyruvate is reduced, so that the binding strength between the cell wall 4 and the outer membrane 5 is weakened, and the outer membrane 5 is likely to be partially detached from the cell wall 4. This promotes the leakage of the protein and the metabolite produced in the bacterial cell from the bacterial cell and the leakage of the plant disease resistance inducer produced in the bacterial cell from the bacterial cell.

As described above, it is thought that a gene encoding a protein with a base sequence identity of 30% or more is likely to express a protein with a function equivalent to that of the protein. Thus, the gene encoding the cell-wall-pyruvate modification enzyme 9 with a suppressed or eliminated function may be, for example, a gene that has a base sequence identity of 40% or more, preferably 50% or more, more preferably 60% or more, still more preferably 70% or more, still more preferably 80% or more, still more preferably 90% or more, with the base sequence of any of genes encoding the cell-wall-pyruvate modification enzymes 9 represented by SEQ ID Nos. 10 to 12, and that encodes a protein or polypeptide having a function of catalyzing the reaction of modifying the covalently linked sugar chain 3 of the peptidoglycan 2 of the cell wall 4 with a pyruvate.

### [5. Method for Producing Modified Cyanobacterium]

Next, a method for producing the modified cyanobacterium according to the present embodiment is described. The method for producing the modified cyanobacterium includes a step of suppressing or eliminating the function of a protein involved in the binding between the outer membrane 5 and the cell wall 4 in the cyanobacterium.

In the present embodiment, the protein involved in the binding between the outer membrane 5 and the cell wall 4 may be, for example, at least one of the SLH-domain-containing outer membrane protein 6 and the cell-wall-pyruvate modification enzyme 9.

The means for suppressing or eliminating the function of the protein may be, but is not limited to, for example, to delete or inactivate a gene encoding the SLH-domain-containing outer membrane protein 6 and a gene encoding the cell-wall-pyruvate modification enzyme 9, to inhibit the transcription of these genes, to inhibit the translation of transcription products of these genes, or to administer an inhibitor that specifically inhibits these proteins.

The means for deleting or inactivating a gene may be, for example, introduction of a mutation into one or more bases in the base sequence of the gene, substitution of the base sequence with another base sequence or insertion of another base sequence into the base sequence, or deletion of part or all of the base sequence of the gene.

The means for inhibiting transcription of the gene may be, for example, introduction of a mutation into the promoter region of the gene, inactivation of the promoter by substitution with another base sequence or insertion of another base sequence, or a CRISPR interference method (Non-Patent Literature 7: Yao et al., ACS Synth. Biol., 2016, 5: 207-212). A specific method for the introduction of a mutation, the substitution with a base sequence, or the insertion of a base sequence may be, for example, ultraviolet irradiation, site-directed mutagenesis, or homologous recombination.

The means for inhibiting the translation of transcription products of the genes may be, for example, a ribonucleic acid (RNA) interference method.

Any of these means may be used to suppress or eliminate the function of a protein involved in the binding between the outer membrane 5 and the cell wall 4 in a cyanobacterium to produce a modified cyanobacterium.

This partially reduces the binding between the cell wall 4 and the outer membrane 5 (that is, the amount and strength of binding) in a modified cyanobacterium produced by the production method, and the outer membrane 5 is therefore likely to be partially detached from the cell wall 4. Consequently, in the modified cyanobacterium, an intracellular product, such as a protein or a metabolite produced in a bacterial cell, is likely to leak out of the outer membrane 5 (that is, out of the bacterial cell), and a substance involved in plant disease resistance induction (that is, a plant disease resistance inducer) is also likely to leak out of the bacterial cell. Thus, the method for producing the modified cyanobacterium according to the present embodiment can provide a modified cyanobacterium with improved secretory productivity of a plant disease resistance inducer.

In a modified cyanobacterium produced by the production method according to the present embodiment, a plant disease resistance inducer produced in a bacterial cell leaks out of the bacterial cell, and it is therefore not necessary to disrupt the bacterial cell to recover the plant disease resistance inducer. For example, a modified cyanobacterium may be cultured under appropriate conditions, and then a plant disease resistance inducer secreted into a culture fluid may be recovered. Thus, it is also possible to recover the plant disease resistance inducer in the culture fluid while culturing the modified cyanobacterium. Thus, a modified cyanobacterium produced by the present production method can be used to efficiently produce a microbial plant disease resistance inducer. Thus, the method for producing the modified cyanobacterium according to the present embodiment can provide a modified cyanobacterium with high use efficiency that can be repeatedly used even after a plant disease resistance inducer is recovered.

### [6. Plant Disease Resistance Inducing Method]

A plant disease resistance inducing method according to the present embodiment uses the plant disease resistance inducing agent. As described above, the plant disease resistance inducing agent according to the present embodiment is a plant disease resistance inducing agent with an improved plant disease resistance inducing effect and can therefore be used to effectively induce plant disease resistance.

The plant disease resistance inducing agent may be used as it is or may be concentrated or diluted before use. When the plant disease resistance inducing agent is applied to a plant, the concentration and the application method of the plant disease resistance inducing agent may be appropriately determined depending on the type of the plant, the properties of soil, the purpose, and the like. The plant disease resistance inducing agent may be, for example, a culture fluid itself of a modified cyanobacterium, a solution produced by removing bacterial cells of the modified cyanobacterium from the culture fluid, or an extract from the culture fluid using a membrane technique or the like. The dosage form of the plant disease resistance inducing agent may be a liquid form or a powder form produced by drying a liquid plant disease resistance inducing agent, for example, by spray drying or another technique. The method for applying the plant disease resistance inducing agent to a plant may be, for example, spraying, irrigation, mixing, or the like to the plant or soil. More specifically, for example, several milliliters per plant body may be added to the root of the plant body or may be sprayed on leaves approximately once a week.

### EXAMPLES

A modified cyanobacterium, a method for producing a modified cyanobacterium, a plant disease resistance inducing agent, a plant disease resistance inducing method, and a method for producing a plant disease resistance inducing agent according to the present disclosure are more specifically described in the following examples. However, the present disclosure is not limited to these examples.

In the following examples, two types of modified cyanobacteria were produced by repressing the expression of an slr1841 gene encoding a SLH-domain-containing outer membrane protein (Example 1) and repressing the expression of an slr0688 gene encoding a cell-wall-pyruvate modification enzyme (Example 2) as a method for partially detaching an outer membrane of a cyanobacterium from a cell wall. The secretory productivity of proteins of these modified cyanobacteria was measured, and a secreted intracellular product (a protein or an intracellular metabolite) was identified. The cyanobacterium used in the present example is Synechocystis sp. PCC 6803 (hereinafter referred to simply as a "cyanobacterium").

### (Example 1)

In Example 1, a modified cyanobacterium was produced in which the expression of an slr1841 gene encoding a SLH-domain-containing outer membrane protein was repressed.

### (1) Construction of Cyanobacterium Modified Strain with Repressed Expression of slr1841 Gene

A clustered regularly interspaced short palindromic repeat (CRISPR) interference method was used as a method for repressing the expression of a gene. In this method, a gene encoding a dCas9 protein (hereinafter referred to as a dCas9 gene) and slr1841_sgRNA (single-guide ribonucleic acid) gene can be introduced into a chromosomal DNA of a cyanobacterium to repress the expression of an slr1841 gene.

The mechanism of the repression of gene expression by the present method is described below.

First, a Cas9 protein deficient in nuclease activity (dCas9) and sgRNA (slr1841_sgRNA) complementarily binding to the base sequence of the slr1841 gene form a complex.

This complex then recognizes the slr1841 gene in a chromosomal DNA of a cyanobacterium and binds specifically to the slr1841 gene. This binding causes steric hindrance and inhibits transcription of the slr1841 gene. This represses the expression of the slr1841 gene of the cyanobacterium.

A method for introducing each of the two genes into a chromosomal DNA of a cyanobacterium is more specifically described below.

### (1-1) Introduction of dCas9 Gene

Using a chromosomal DNA of a Synechocystis LY07 strain (hereinafter also referred to as an LY07 strain) (see Non-Patent Literature 7) as a template, the dCas9 gene, an operator gene for controlling the expression of the dCas9 gene, and a spectinomycin-resistant marker gene serving as a marker for gene transfer were amplified by a polymerase chain reaction (PCR) method using primers psbA1-Fw (SEQ ID No. 13) and psbA1-Rv (SEQ ID No. 14) shown in Table 1. In the LY07 strain, the three genes in a linked state are inserted into a psbA1 gene in the chromosomal DNA and can be amplified as one DNA fragment by the PCR method. The DNA fragment is referred to as a "psbA1::dCas9 cassette". Using an In-Fusion PCR cloning method (registered trademark), the psbA1:: dCas9cassette was inserted into a pUC19 plasmid to prepare a pUC19-dCas9 plasmid.

**[Table 1]**

| Primer name | Base sequence | SEQ ID No. |
|---|---|---|
| psbA1-Fw | 5'-CAGTGAATTCGAGCTCGGTATATAGCGTTGCAGTCCCTGG-3' | 13 |
| psbA1-Rv | 5'-GATTACGCCAAGCTTGCATGACCGCGGTCACTTCATAACC-3' | 14 |
| slr2030-Fw | 5'-CAGTGAATTCGAGCTCGGTAATAACCGTTGTCCCTTTTGTTTCATCG-3' | 15 |
| sgRNA_slr1841-Rv | 5'-TGTTAGTGAGCCCTGCTGTTAGCTCCCAGTATCTCTATCACTGAT-3' | 16 |
| sgRNA_slr1841-Fw | 5'-ACAGCAGGGCTCACTAACAGTTTTAGAGCTAGAAATAGCAAGTTAAAATAA-3' | 17 |
| slr2031-Rv | 5'-GATTACGCCAAGCTTGCATGGGGAACAAGCTGAATCTGGGCATC-3' | 18 |
| sgRNA_slr0688-Rv | 5'-TTTTAGTCTGTTTGCTGCATAGCTCCCAGTATCTCTATCACTGAT-3' | 19 |
| sgRNA_slr0688-Fw | 5'-TGCAGCAAACAGACTAAAAGTTTTAGAGCTAGAAATAGCAAGTTAAAATAA-3' | 20 |

1 µg of the pUC19-dCas9 plasmid was mixed with a cyanobacterium culture fluid (bacterial cell concentration OD730 = approximately 0.5), and the pUC19-dCas9 plasmid was introduced into a cell of the cyanobacterium by a spontaneous transformation method. The transformed cell was selected by growth on a BG-11 agar medium containing 20 µg/mL spectinomycin. In the selected cell, homologous recombination has occurred between the psbA1 gene in the chromosomal DNA and the psbA1 upstream fragment region and the psbA1 downstream fragment region in the pUC19-dCas9 plasmid. Thus, a Synechocystis dCas9 strain with a dCas9 cassette inserted into a psbA1 gene region was prepared. The composition of the BG-11 culture medium used is shown in Table 2.

**[Table 2]**

| Component | Content (mg/L) |
|---|---|
| EDTA-Na | 1 |
| Ammonium Iron Citrate | 6 |
| NaNO₃ | 1500 |
| MgSO₄ | 75 |
| K₂HPO₄ | 39 |
| CaCl₂ | 28.6 |
| H₃BO₄ | 2.8 |
| MnCl₂ | 1.8 |
| ZnSO₄ | 0.2 |
| CuSO₄ | 0.08 |
| Na₂MoO₄ | 0.02 |
| Co(NO₃)₂ | 0.005 |
| TES-KOH (pH 7.5) | 4580 |

### (1-2) Introduction of slr1841_sgRNA Gene

In the CRISPR interference method, sgRNA binds specifically to a target gene by introducing a sequence of approximately 20 bases complementary to the target sequence into a region called a protospacer in the sgRNA gene. Table 3 shows protospacer sequences used in the present example.

**[Table 3]**

| Protospacer target gene | Base sequence | SEQ ID No. |
|---|---|---|
| slr1841 | 5'-ACAGCAGGGCTCACTAACA-3' | 21 |
| slr0688 | 5'-TGCAGCAAACAGACTAAAA-3' | 22 |

In the Synechocystis LY07 strain, the sgRNA gene (excluding the protospacer region) and a kanamycin-resistant marker gene are linked and inserted into slr2030-slr2031 genes in the chromosomal DNA. Thus, sgRNA (slr1841_sgRNA) that specifically recognizes slr1841 can be easily produced by adding a protospacer sequence (SEQ ID No. 21) complementary to the slr1841 gene (SEQ ID No. 7) to a primer used to amplify the sgRNA gene by the PCR method.

First, two DNA fragments were amplified by the PCR method using the chromosomal DNA of the LY07 strain as a template and using a set of primers slr2030-Fw (SEQ ID No. 15) and sgRNA_slr1841-Rv (SEQ ID No. 16) and a set of sgRNA_slr1841-Fw (SEQ ID No. 17) and slr2031-Rv (SEQ ID No. 18) shown in Table 1.

Subsequently, a DNA fragment (slr2030-2031::slr1841_sgRNA) including (i) an slr2030 gene fragment, (ii) slr1841_sgRNA, (iii) a kanamycin-resistant marker fragment, and (iv) an slr2031gene fragment linked in this order was produced by a PCR amplification method using a mixed solution of the DNA fragments as a template and using the primers slr2030-Fw (SEQ ID No. 15) and slr2031-Rv(SEQ ID No. 18) shown in Table 1. Using the In-Fusion PCR cloning method (registered trademark), the slr2030-2031::slr1841_sgRNA was inserted into the pUC19 plasmid to prepare a pUC19-slr1841_sgRNA plasmid.

The pUC19-slr1841_sgRNA plasmid was introduced into the Synechocystis dCas9 strain in the same manner as in (1-1), and the transformed cell was selected on a BG-11 agar medium containing 30 µg/mL of kanamycin. Thus, a transformant Synechocystis dCas9 slr1841_sgRNA strain (hereinafter also referred to as an slr1841 repressed strain) with slr1841_sgRNA inserted into the slr2030-slr2031 genes in the chromosomal DNA was prepared.

### (1-3) Repression of slr1841 Gene

For the dCas9 gene and the slr1841_sgRNA gene, the promoter sequence is designed so that their expression is induced in the presence of anhydrotetracycline (aTc). In the present example, aTc was added to the culture medium at a final concentration of 1 µg/mL to repress the expression of the slr1841 gene.

### (Example 2)

In Example 2, a modified cyanobacterium in which the expression of the slr0688 gene encoding the cell-wall-pyruvate modification enzyme was repressed was produced by the following procedure.

### (2) Construction of Cyanobacterium Modified Strain with Repressed Expression of slr0688 Gene

In the same manner as in (1-2), an sgRNA gene containing a protospacer sequence (SEQ ID No. 22) complementary to the slr0688 gene (SEQ ID No. 4) was introduced into the Synechocystis dCas9 strain to prepare a Synechocystis dCas9 slr0688_sgRNA strain. It was performed under the same conditions as in (1-2) except that a set of primers slr2030-Fw (SEQ ID No. 15) and sgRNA_slr0688-Rv (SEQ ID No. 19) and a set of sgRNA_slr0688-Fw (SEQ ID No. 20) and slr2031-Rv (SEQ ID No. 18) shown in Table 1 were used, and a DNA fragment (slr2030-2031::slr0688_sgRNA) in which (i) the slr2030 gene fragment, (ii) an slr0688_sgRNA, (iii) a kanamycin-resistant marker gene, and (iv) an slr2031 gene fragment were linked in this order was inserted into the pUC19 plasmid by the In-Fusion PCR cloning method (registered trademark) to prepare a pUC19-slr0688_sgRNA plasmid.

Furthermore, the expression of the slr0688 gene was repressed in the same manner as in (1-3).

### (Comparative Example 1)

In Comparative Example 1, a Synechocystis dCas9 strain was prepared in the same manner as in (1-1) of Example 1.

Subsequently, for each strain prepared in Example 1, Example 2, and Comparative Example 1, the state of the cell surface layer was observed, and a protein secretory productivity test was performed. The details are described below.

### (3) Observation of State of Cell Surface Layer of Strain

Ultrathin sections of the modified cyanobacterium Synechocystis dCas9 slr1841_sgRNA strain prepared in Example 1 (that is, an slr1841 repressed strain), the modified cyanobacterium Synechocystis dCas9slr0688_sgRNA strain prepared in Example 2 (hereinafter also referred to as an slr0688 repressed strain), and the modified cyanobacterium Synechocystis dCas9 strain prepared in Comparative Example 1 (hereinafter referred to as Control strains) were prepared, and the state of the cell surface layer (in other words, the outer membrane structure) was observed with an electron microscope.

### (3-1) Culture of Strain

The slr1841 repressed strain of Example 1 was inoculated into a BG-11 culture medium containing 1 µg/mL aTc at an initial bacterial cell concentration OD730 = 0.05 and was cultured with shaking at a light intensity of 100 µmol/m²/s at 30°C for 5 days. The slr0688 repressed strain of Example 2 and the Control strain of Comparative Example 1 were also cultured under the same conditions as in Example 1.

### (3-2) Preparation of Ultrathin Section of Strain

The culture fluid prepared in (3-1) was centrifuged at room temperature at 2,500 g for 10 minutes to recover cells of the slr1841 repressed strain of Example 1. The cells were then rapidly frozen in liquid propane at -175°C and was then fixed with an ethanol solution containing 2% glutaraldehyde and 1% tannic acid at -80°C for 2 days. The fixed cells were dehydrated with ethanol, were infiltrated with propylene oxide, and were then submerged in a resin (Quetol-651) solution. The resin solution was then allowed to stand at 60°C for 48 hours to cure the resin and embed the cells in the resin. The cells in the resin were sliced to a thickness of 70 nm using an ultramicrotome (Ultracut) to prepare an ultrathin section. The ultrathin section was stained with a 2% uranium acetate and 1% lead citrate solution to prepare a sample of the slr1841 repressed strain of Example 1 for a transmission electron microscope. Samples of the slr0688 repressed strain of Example 2 and the Control strain of Comparative Example 1 for a transmission electron microscope were also prepared in the same manner.

### (3-3) Observation with Electron Microscope

The ultrathin sections prepared in (3-2) were observed with a transmission electron microscope (JEOL JEM-1400Plus) at an accelerating voltage of 100 kV. Figs. 3 to 8 show the observation results.

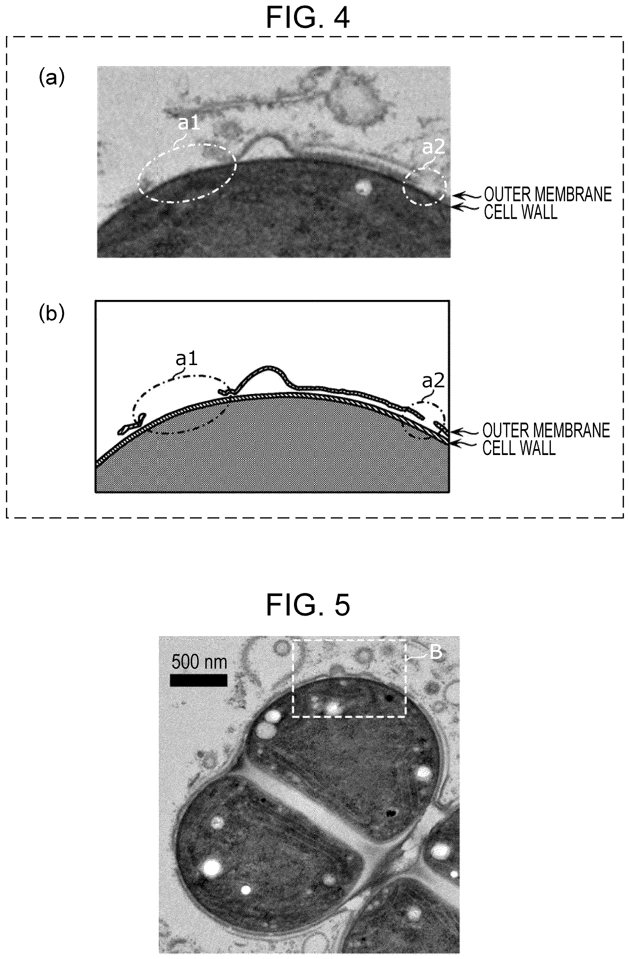

First, the slr1841 repressed strain of Example 1 is described. Fig. 3 is a transmission electron microscope (TEM) image of the slr1841 repressed strain of Example 1. Fig. 4 is a magnified image of a broken-line region A in Fig. 3. Fig. 4(a) is a magnified TEM image of the broken-line region A in Fig. 3, and Fig. 4(b) is a view illustrating the magnified TEM image of Fig. 4(a).

As shown in Fig. 3, in the slr1841 repressed strain of Example 1, the outer membrane was partially separated from the cell wall (that is, the outer membrane was partially peeled off) and was partially bent.

When the broken-line region A was observed under magnification to examine the state of the cell surface layer in more detail, as shown in Figs. 4(a) and 4(b), portions where the outer membrane was partially peeled off (dash-dotted line regions a1 and a2 in the drawings) were observed. Furthermore, a portion where the outer membrane was largely bent was observed near the dash-dotted line region a1. It is thought that the binding between the outer membrane and the cell wall was weakened in this portion, and the culture fluid permeated from the outer membrane into the periplasm outwardly expanded and bent the outer membrane.

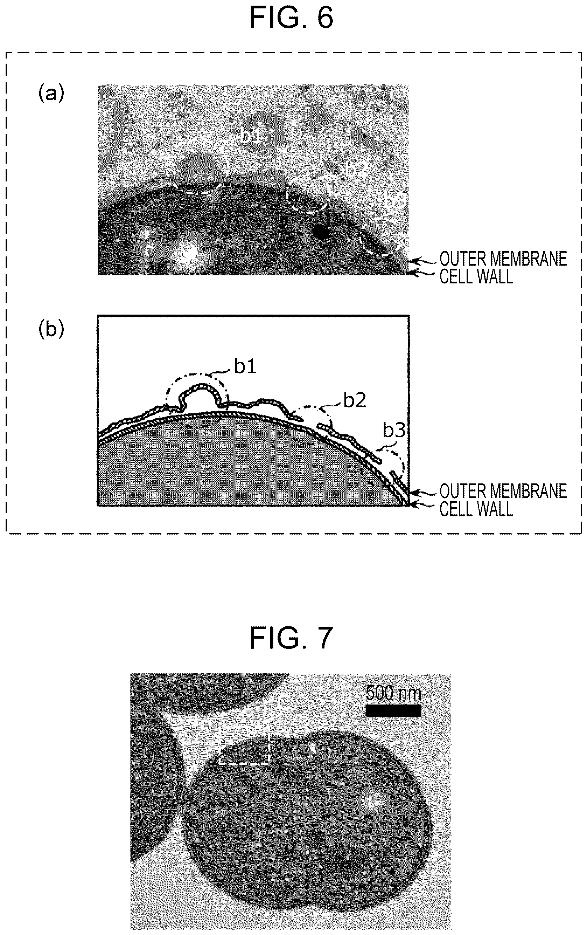

Next, the slr0688 repressed strain of Example 2 is described. Fig. 5 is a TEM image of the slr0688 repressed strain of Example 2. Fig. 6 is a magnified image of a broken-line region B in Fig. 5. Fig. 6(a) is a magnified TEM image of the broken-line region B in Fig. 5, and Fig. 6(b) is a view illustrating the magnified TEM image of Fig. 6(a).

As shown in Fig. 5, in the slr0688 repressed strain of Example 2, the outer membrane was partially separated from the cell wall and was partially bent. It was also shown that the outer membrane was partially detached from the cell wall in the slr0688 repressed strain.

When the broken-line region B was observed under magnification to examine the state of the cell surface layer in more detail, as shown in Figs. 6(a) and 6(b), a portion where the outer membrane was largely bent (a dash-dotted line region b1 in the drawing) and a portion where the outer membrane was partially peeled off (dash-dotted line regions b2 and b3 in the drawing) were observed. Furthermore, a portion where the outer membrane was detached from the cell wall was observed near each of the dash-dotted line regions b1, b2, and b3.

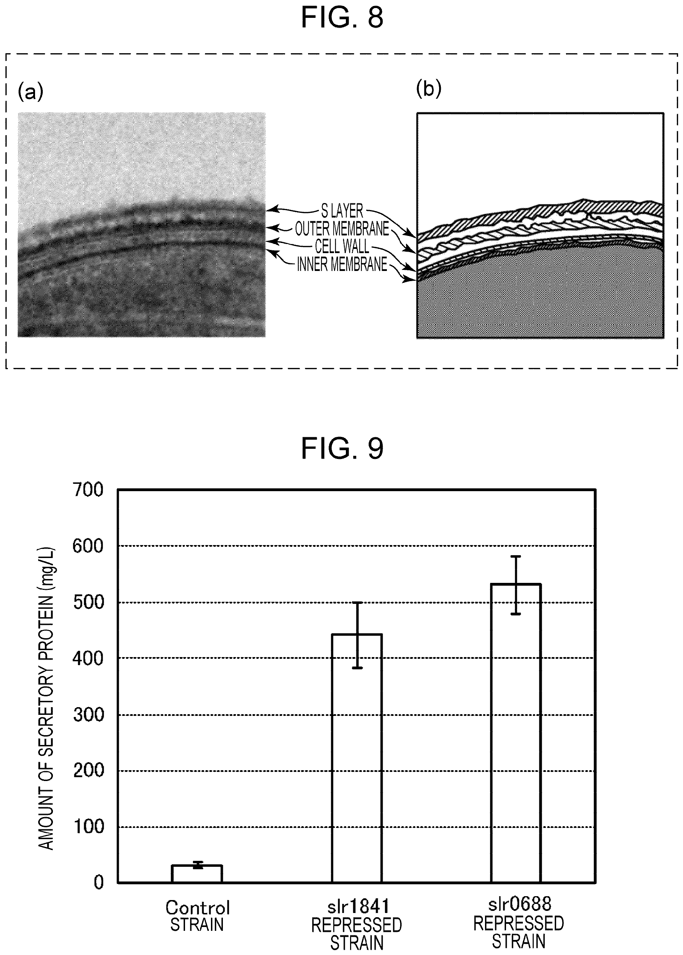

Next, the Control strain of Comparative Example 1 is described. Fig. 7 is a TEM image of the Control strain of Comparative Example 1. Fig. 8 is a magnified image of a broken-line region C in Fig. 7. Fig. 8(a) is a magnified TEM image of the broken-line region C in Fig. 7, and Fig. 8(b) is a view illustrating the magnified TEM image of Fig. 8(a).

As shown in Fig. 7, the cell surface layer of the Control strain of Comparative Example 1 was uniform, and the inner membrane, the cell wall, the outer membrane, and an S layer were stacked in this order. Thus, in the Control strain, a portion where the outer membrane was detached from the cell wall, a portion where the outer membrane was separated (that is, peeled off) from the cell wall, and a portion where the outer membrane was bent as in Examples 1 and 2 were not observed.

### (4) Protein Secretory Productivity Test

The slr1841 repressed strain of Example 1, the slr0688 repressed strain of Example 2, and the Control strain of Comparative Example 1 were cultured, and the amount of protein secreted from cells (hereinafter also referred to as the amount of secretory protein) was measured. The protein secretory productivity of each strain was evaluated based on the amount of protein in the culture fluid. The protein secretory productivity refers to the ability to produce a protein by secreting the protein produced in a cell from the cell. A specific method is described below.

### (4-1) Culture of Strain

The slr1841 repressed strain of Example 1 was cultured in the same manner as in (3-1). The culture was independently performed three times. The strains of Example 2 and Comparative Example 1 were also cultured under the same conditions as in the strain of Example 1.

### (4-2) Quantitative Determination of Protein Secreted from Cell

The culture fluid prepared in (4-1) was centrifuged at room temperature at 2,500 g for 10 minutes to prepare a culture supernatant. The culture supernatant was filtered through a membrane filter with a pore size of 0.22 µm to completely remove the cells of the slr1841 repressed strain of Example 1. The total protein in the culture supernatant after the filtration was determined by a bicinchoninic acid (BCA) method. This series of operations was performed for each of the three independently cultured culture fluids to determine the average value and standard deviation of the amount of protein secreted from the cells of the slr1841 repressed strain of Example 1. For each strain of Example 2 and Comparative Example 1, the amount of protein in the three culture fluids was also determined under the same conditions, and the average value and standard deviation of the amount of protein in the three culture fluids were determined.

Fig. 9 shows the results. Fig. 9 is a graph of the amount of protein in the culture supernatants of the modified cyanobacteria of Example 1, Example 2, and Comparative Example 1 (n = 3, error bar = SD).

As shown in Fig. 9, the amount of protein (mg/L) secreted into the culture supernatant in the slr1841 repressed strain of Example 1 and the slr0688 repressed strain of Example 2 was approximately 25 times that in the Control strain of Comparative Example 1.

Although data are not shown, the absorbance (730 nm) of the culture fluid was measured to calculate the amount of secretory protein per gram of dried bacterial cell (mg protein/g cell dry weight). As a result, the amount of secretory protein per gram of dried bacterial cell (mg protein/g cell dry weight) in the slr1841 repressed strain of Example 1 and the slr0688 repressed strain of Example 2 was approximately 36 times that in the Control strain of Comparative Example 1.

As shown in Fig. 9, the amount of protein secreted into the culture supernatant was larger in the slr0688 repressed strain of Example 2 in which the expression of the gene encoding the cell-wall-pyruvate modification enzyme (slr0688) was repressed than in the slr1841 repressed strain of Example 1 in which the expression of the gene encoding the SLH-domain-containing outer membrane protein (slr1841) was repressed. This is considered to be related to the fact that the number of covalently linked sugar chains on the surface of the cell wall is larger than the number of SLH-domain-containing outer membrane proteins (Slr1841) in the outer membrane. In other words, it is thought that the slr0688 repressed strain of Example 2 was lower in the amount and strength of binding between the outer membrane and the cell wall than the slr1841 repressed strain of Example 1 and was therefore larger in the amount of secretory protein than the slr1841 repressed strain of Example 1.

The results showed that suppressing the function of the protein associated with the binding between the outer membrane and the cell wall partially weakened the binding between the outer membrane and the cell wall of cyanobacterium and partially detached the outer membrane from the cell wall. It was also shown that the weakening of the binding between the outer membrane and the cell wall facilitated the leakage of the protein produced in a cell of the cyanobacterium from the cell. Thus, it was shown that the modified cyanobacterium and the method for producing the modified cyanobacterium according to the present embodiment greatly improved the protein secretory productivity.

### (5) Identification of Secretory Protein

Subsequently, the secretory protein in the culture supernatant prepared in (4-2) was identified by LC-MS/MS. The method is described below.

### (5-1) Sample Preparation

Cold acetone was added in an amount of 8 times the amount of the culture supernatant, and the mixture was allowed to stand at 20°C for 2 hours and was then centrifuged at 20,000 g for 15 minutes to obtain a precipitate of the protein. 100 mM Tris pH 8.5 and 0.5% sodium dodecanoate (SDoD) were added to the precipitate, and the protein was dissolved with a sealed ultrasonic homogenizer. After adjusting the protein concentration to 1 µg/mL, dithiothreitol (DTT) was added at a final concentration of 10 mM, and the mixture was allowed to stand at 50°C for 30 minutes. Iodoacetamide (IAA) was added at a final concentration of 30 mM, and the mixture was allowed to stand at room temperature (shaded) for 30 minutes. To stop the reaction of IAA, cysteine was added at a final concentration of 60 mM, and the mixture was allowed to stand at room temperature for 10 minutes. 400 ng of trypsin was added, and the mixture was allowed to stand at 37°C overnight to fragment the protein into peptides. After adding 5% trifluoroacetic acid (TFA), the mixture was centrifuged at room temperature at 15,000 g for 10 minutes to prepare a supernatant. SDoD was removed by this operation. After desalination with a C18 spin column, the sample was dried with a centrifugal evaporator. 3% acetonitrile and 0.1% formic acid were then added, and the sample was dissolved with the sealed ultrasonic homogenizer. The peptide concentration was adjusted to 200 ng/µL.

### (5-2) LC-MS/MS Analysis

The sample prepared in (5-1) was analyzed with a LC-MS/MS apparatus (UltiMate 3000 RSLCnano LC System) under the following conditions.

Sample injection amount: 200 ng
Column: CAPCELL CORE MP 75 µm x 250 mm
Solvent: A solvent was 0.1% aqueous formic acid, and B solvent was 0.1% formic acid + 80% acetonitrile
Gradient program: 8% B solvent 4 minutes after sample injection, 44% B solvent 27 minutes later, 80% B solvent 28 minutes later, end of measurement 34 minutes later.

### (5-3) Data Analysis

The obtained data were analyzed under the following conditions to identify proteins and peptides and calculate quantitative values.

Software: Scaffold DIA
Database: UniProtKB/Swiss Prot database (Synechocystis sp. PCC 6803)
Fragmentation: HCD Precursor Tolerance: 8 ppm
Fragment Tolerance: 10 ppm
Data Acquisition Type: Overlapping DIA
Peptide Length: 8-70
Peptide Charge: 2-8
Max Missed Cleavages: 1
Fixed Modification: Carbamidomethylation
Peptide FDR: 1% or less

Table 4 shows proteins that are expected to have significant enzyme activity among 30 proteins identified with the highest relative quantitative values.

**[Table 4]**

| | Protein name | Uniprot Accession ID | Gene name |
|---|---|---|---|
| 1 | Carboxyl-terminal protease | P73458 | prc |
| 2 | Iron uptake protein A2 | Q55835 | futA2 |
| 3 | Extracellular nuclease | P72938 | nucH |
| 4 | Alkaline phosphatase | P72939 | sl10654 |
| 5 | N-acetylmuramoyl-L-alanine amidase | P73736 | amiA |
| 6 | Peptidyl-prolyl cis-trans isomerase | P73037 | ytfC |

All of these six proteins were contained in each of the culture supernatants of the slr1841 repressed strain of Example 1 and the slr0688 repressed strain of Example 2. In all of these proteins, the periplasmic (referring to the space between the outer membrane and the inner membrane) localization signal was retained. This result showed that, in the modified strains of Examples 1 and 2, the outer membrane was partially detached from the cell wall, and a protein in the periplasm was likely to leak out of the outer membrane (that is, out of the bacterial cell). Thus, it was shown that the modified cyanobacterium according to the present embodiment has significantly improved protein secretory productivity.

### (6) Identification of Secreted Intracellular Metabolite

### (6-1) Sample Preparation

20 µl of an aqueous solution with an internal standard substance concentration adjusted to 1,000 µM was added to 80 µl of the culture supernatant of the modified cyanobacterium, and the mixture was stirred, ultrafiltered, and then subjected to measurement.

### (6-2) Capillary Electrophoresis (CE)-Time-Of-Flight Mass Spectrometry (TOFMS) Analysis

In this test, measurements in the cation mode and the anion mode were performed under the following conditions.

### [Cation Mode]

Apparatus: Agilent CE-TOFMS system
Capillary: Fused silica capillary i.d. 50 µm x 80 cm

### Measurement conditions:

Run buffer: Cation buffer solution (p/n: H3301-1001)
CE voltage: Positive, 30 kV
MS ionization: ESI positive
MS scan range: m/z 50-1,000

### [Anion Mode]

Apparatus: Agilent CE-TOFMS system
Capillary: Fused silica capillary i.d. 50 µm x 80cm

### Measurement conditions:

Run buffer: Anion buffer solution (p/n: H3301-1001)
CE voltage: Positive, 30 kV
MS ionization: ESI negative
MS scan range: m/z 50-1,000

### (6-3) Data Processing

Among peaks detected by CE-TOFMS, peaks with a signal/noise ratio of 3 or more were automatically detected using automatic integration software MasterHands (registered trademark) ver. 2.17.1.11. Metabolites in the culture supernatant of the modified cyanobacterium were searched for by comparing the detected peaks with the values of all substances registered in the metabolite library of Human Metabolome Technologies, Inc. (HMT) based on the mass-to-charge ratio (m/z) and migration time specific to each metabolite. The tolerances for the search were +/-0. 5 min for the migration time and +/-10 ppm for m/z. The concentration of each identified metabolite was calculated using a single-point calibration at 100 µM. Table 5 shows major metabolites identified.

**[Table 5]**

| | Metabolite name | Concentration (µM) | |
|---|---|---|---|
| | | slr1841 suppressor strain | slr0688 suppressor strain |
| 1 | 3-hydroxybutyric acid | 1.3 | 1.2 |
| 2 | adenosine | 0.8 | 0.6 |
| 3 | aspartic acid | 4.6 | 3.0 |
| 4 | cytidine | 0.7 | 0.5 |
| 5 | guanosine | 1.5 | 1.1 |
| 6 | Gluconic acid | 2.7 | 3.9 |
| 7 | Glutathione (GSSG) | 0.4 | 0.4 |
| 8 | Lactic acid | 3.2 | 4.7 |
| 9 | Malic acid | 1.4 | 2.8 |
| 10 | p-aminobeizoic acid | 1.7 | 0.9 |
| 11 | S-adenosylmethionine | 0.2 | 0.1 |
| 12 | spermidine | 4.2 | 3.0 |

All of these 12 intracellular metabolites were contained in each of the culture supernatants of the slr1841 repressed strain of Example 1 and the slr0688 repressed strain of Example 2. Although data are not shown, the culture supernatant of the Control strain of Comparative Example 1 did not contain these metabolites. This result showed that, in the modified strains of Examples 1 and 2, the outer membrane was partially detached from the cell wall, and an intracellular metabolite was likely to leak out of the outer membrane (that is, out of the bacterial cell).

### (7) Disease Resistance Induction Test

Subsequently, the following plant cultivation test and gene expression study were performed to evaluate the plant disease resistance effect of a secretion product of the modified cyanobacterium (here, the culture supernatant of the modified cyanobacterium). In the gene expression study, the effect of a plant disease resistance inducing agent was evaluated by examining the expression variation of a gene involved in disease resistance in a plant cultivated in the plant cultivation test. The modified cyanobacterium was the slr0688 repressed strain of Example 2, and a strain was independently cultured three times in the same manner as in (4-1). Thus, in Examples 3 and 4, the culture supernatant of the modified cyanobacterium of Example 2 was used as a plant disease resistance inducing agent.

### (7-1) Plant Cultivation Test

### (7-1-1) Tomato Cultivation Test

In the tomato cultivation test, cherry tomatoes were cultivated by the following method. First, a commercially available culture soil was placed in a cultivation planter (22 cm x 16 cm), and three tomato seeds per planter were sown. Cultivation was performed under conditions of an indoor temperature of 23°C, a photon flux density of a white light source of 250 µmol/m²/s, a light condition of 16 hours, and a dark condition of 8 hours. When cotyledons developed approximately one week after the start of cultivation, the individual size in each planter was made uniform by thinning. Furthermore, 500 mL of a commercially available chemical fertilizer (a 500-fold diluted solution of a stock solution containing 6% of nitrogen in total, 10% of water-soluble phosphoric acid, 5% of water-soluble potassium, 0.05% of water-soluble magnesium oxide, 0.001% of water-soluble manganese, and 0.005% of water-soluble boron) was applied to each planter once every 50 days.

### (Example 3)

In Example 3, as described above, the individual size in each planter was made uniform, and after planting of cherry tomato seedlings, the plant disease resistance inducing agent diluted 20-fold with water was sprayed on leaves once every two weeks in an amount of 10 mL per tomato plant.

### (Comparative Example 2)

Comparative Example 2 was performed in the same manner as in Example 3 except that water was used instead of the disease resistance inducing agent.

### (7-1-2) Spinach Cultivation Test

First, a commercially available culture soil was placed in a cultivation pot (12 cm x 10 cm), and three spinach seeds per pot were sown. Cultivation was performed under conditions of an indoor temperature of 23°C, a photon flux density of a white light source of 100 µmol/m²/s, a light condition of 10 hours, and a dark condition of 14 hours. When cotyledons developed approximately one week after the start of cultivation, the individual size in each pot was made uniform by thinning.

### (Example 4)

In Example 4, as described above, the individual size in each pot was made uniform, and two weeks after sowing, the plant disease resistance inducing agent diluted 20-fold with water was sprayed on leaves such that the leaves were visually wetted.

### (Comparative Example 3)

Comparative Example 3 was performed in the same manner as in Example 4 except that water was used instead of the disease resistance inducing agent.

### (7-2) Gene Expression Study

For the cherry tomatoes cultivated in Example 3 and Comparative Example 2, leaves were cut off 100 days after sowing, and for the spinach cultivated in Example 4 and Comparative Example 3, leaves were cut off 20 days after sowing, and Total RNA was prepared using a Nucleospin (registered trademark) RNA plant kit (manufactured by Takara Bio Inc.). The Total RNA was treated with TruSeq (registered trademark) Standard mRNA LT Sample Prep Kit (manufactured by Illumina) to prepare a library, and the prepared library was subjected to transcriptome sequencing using a NovaSeq (registered trademark) next-generation sequencer (manufactured by Illumina). More specifically, samples of a comparative example (an untreated group) and an example (a treated group) were subjected to RNA-Seq using the next-generation sequencer to detect transcripts (genes) with different expression levels in the untreated group and the treated group. The expression levels of Nonexpressor of Pathogenesis-Related 1 (NPR1) genes, TGA genes, and Pathogenesis-Related-1 (PR-1) genes, which are widely present in plants as genes involved in plant disease resistance (hereinafter referred to as plant disease resistance related genes), among the detected genes were then analyzed to investigate the expression variation of the plant disease resistance related genes.

### (Results)

Table 6 shows the results of the expression level analysis of the plant disease resistance related genes of Examples 3 and 4. Table 7 shows the NCBI-gene IDs of disease resistance genes corresponding to *1 to *8 shown in Table 6.

**[Table 6]**

| | NPR1 gene | TGA gene | PR-1 gene |
|---|---|---|---|
| Example 3 | 2.02*¹ | 14.85*² | 2.02*³ |
| Example 4 | 2.19*⁴ | 4.25*⁵ | 3.02*⁷ |
| | | 53.01*⁶ | 4.11*⁸ |

**[Table 7]**

| | Gene ID |
|---|---|
| *1 | 101245709 |
| *2 | 110805644 |
| *3 | 110775599 |
| *4 | 110780711 |
| *5 | 110786139 |
| *6 | 110805644 |
| *7 | 110775599 |
| *8 | 110777098 |

Table 6 shows, as a result of the expression level analysis of Example 3, the relative value of the expression level (also referred to as the transcriptional level) of the plant disease resistance related gene of the cherry tomato of Example 3 with respect to the expression level (transcriptional level) of the plant disease resistance related gene of the cherry tomato of Comparative Example 2. Table 6 also shows, as a result of the expression level analysis of Example 4, the relative value of the expression level (transcriptional level) of the plant disease resistance related gene of the spinach cultivated in Example 4 with respect to the expression level (transcriptional level) of the plant disease resistance related gene of the spinach cultivated in Comparative Example 3. Table 6 shows that in the cherry tomato (Example 3) and the spinach (Example 4) to which a plant disease resistance inducing agent according to the present disclosure was applied, the expression levels of the NPR1 gene, the TGA gene, and the PR-1 gene were increased at least 2-fold or more. More specifically, the NPR1 gene expression level was increased 2-fold in the cherry tomato (Example 3) and the spinach (Example 4). The TGA gene expression level was increased nearly 15-fold in the cherry tomato (Example 3) and 4- to 53-fold in the spinach (Example 4). The PR-1 gene 3 expression level was increased 2-fold in the cherry tomato (Example 3) and 3- to 4-fold in the spinach (Example 4).

As described above, it is known that the NPR1 gene, the TGA gene, and the PR-1 gene are widely present in plants and improve disease resistance. These results suggest that a disease resistance inducing agent according to the present disclosure has a general-purpose effect applicable to various plants and remarkably activates a disease resistance related gene. Thus, it was confirmed that a plant disease resistance inducing agent according to the present disclosure can effectively induce plant disease resistance.

### Industrial Applicability

A plant disease resistance inducing agent and a plant disease resistance inducing method according to the present disclosure can effectively induce plant disease resistance and can therefore improve resistance to a plant disease. Furthermore, using a method for producing a plant disease resistance inducing agent according to the present disclosure, a cyanobacterium can be cultured to produce a plant disease resistance inducing agent containing a secretion product of the cyanobacterium, and a plant disease resistance inducing agent can therefore be easily and efficiently produced. Thus, the present disclosure can improve resistance to a plant disease and is expected to improve the production and quality of crops.

### Reference Signs List

- 1: inner membrane
- 2: peptidoglycan
- 3: sugar chain
- 4: cell wall
- 5: outer membrane
- 6: SLH-domain-containing outer membrane protein
- 7: SLH domain
- 8: organic channel protein
- 9: cell-wall-pyruvate modification enzyme

## Claims

1. A plant disease resistance inducing agent comprising a secretion product of a cyanobacterium.

2. The plant disease resistance inducing agent according to claim 1, wherein
the cyanobacterium is a modified cyanobacterium in which a function of a protein involved in binding between an outer membrane and a cell wall is suppressed or eliminated.

3. The plant disease resistance inducing agent according to claim 2, wherein
the protein involved in binding between the outer membrane and the cell wall is at least one selected from the group consisting of a surface layer homology (SLH) domain-containing outer membrane protein or a cell-wall-pyruvate modification enzyme.

4. The plant disease resistance inducing agent according to claim 3, wherein
the SLH-domain-containing outer membrane protein is
Slr1841 consisting of an amino acid sequence of SEQ ID No. 1,
NIES970_09470 consisting of an amino acid sequence of SEQ ID No. 2,
Anacy_3458 consisting of an amino acid sequence of SEQ ID No. 3, or
a protein with an amino acid sequence identity of 50% or more with any of these SLH-domain-containing outer membrane proteins.

5. The plant disease resistance inducing agent according to claim 3, wherein
the cell-wall-pyruvate modification enzyme is
Slr0688 consisting of an amino acid sequence of SEQ ID No. 4,
Synpcc7942_1529 consisting of an amino acid sequence of SEQ ID No. 5,
Anacy_1623 consisting of an amino acid sequence of SEQ ID No. 6, or
a protein with an amino acid sequence identity of 50% or more with any of these cell-wall-pyruvate modification enzymes.

6. The plant disease resistance inducing agent according to claim 1, wherein
the cyanobacterium is a modified cyanobacterium in which a gene that expresses the protein involved in binding between the outer membrane and the cell wall is deleted or inactivated.

7. The plant disease resistance inducing agent according to claim 6, wherein
the gene that expresses the protein involved in binding between the outer membrane and the cell wall is at least one selected from the group consisting of a gene encoding a SLH-domain-containing outer membrane protein and a gene encoding a cell-wall-pyruvate modification enzyme.

8. The plant disease resistance inducing agent according to claim 7, wherein
the gene encoding the SLH-domain-containing outer membrane protein is slr1841 consisting of a base sequence of SEQ ID No. 7,
nies970_09470 consisting of a base sequence of SEQ ID No. 8,
anacy_3458 consisting of a base sequence of SEQ ID No. 9, or
a gene with a base sequence identity of 50% or more with any of these genes.

9. The plant disease resistance inducing agent according to claim 7, wherein
the gene encoding the cell-wall-pyruvate modification enzyme is slr0688 consisting of a base sequence of SEQ ID No. 10,
synpcc7942_1529 consisting of a base sequence of SEQ ID No. 11,
anacy_1623 consisting of a base sequence of SEQ ID No. 12, or
a gene with a base sequence identity of 50% or more with any of these genes.

10. A plant disease resistance inducing method, comprising:
using a plant disease resistance inducing agent containing a secretion product of a cyanobacterium.

11. A method for producing a plant disease resistance inducing agent, the method comprising:
preparing a cyanobacterium; and
causing the cyanobacterium to secrete a secretion product involved in induction of plant disease resistance.
